# EUROPEAN PATENT SPECIFICATION

(11) **EP 2 830 639 B1**
(45) Date of publication and mention of the grant of the patent: **08.05.2019**
(21) Application number: 13770363.3
(22) Date of filing: 09.03.2013
(51) Int. Cl.: C12N 5/077, A61K 35/34, A61K 35/545, A61K 38/18, A61P 9/00

(54) **METHODS AND COMPOSITIONS USING FGF-8 TO ENHANCE CARDIAC REGENERATION AND ATTENUATE ADVERSE CARDIAC REMODELING**
VERFAHREN UND ZUSAMMENSETZUNGEN UNTER VERWENDUNG VON FGF-8 ZUR VERBESSERUNG DER REGENERATION DES HERZENS UND ZUR UNTERDRÜCKUNG KARDIALER REMODELLIERUNG
PROCÉDÉS ET COMPOSITIONS À L'AIDE DE FGF-8 POUR AMÉLIORER LA RÉGÉNÉRATION CARDIAQUE ET ATTÉNUER UN REMODELAGE CARDIAQUE DÉFAVORABLE

(30) Priority: 30.03.2012 US 201261617985 P
(43) Date of publication of application: 04.02.2015
(73) Proprietor: University of Central Florida Research Foundation, Inc., Orlando, FL 32826 (US)
(72) Inventor: SINGLA, Dinendar, Orlando, FL 32816 (US)
(74) Representative: Michalski Hüttermann & Partner Patentanwälte mbB
(86) International application number: PCT/US2013/030080
(87) International publication number: WO 2013/148121

(56) References cited:
- EP-A1- 2 098 244
- WO-A1-2009/118928
- WO-A1-2011/139357
- WO-A2-2006/081190
- US-A1- 2002 061 837
- US-A1- 2002 142 457
- US-A1- 2011 097 799
- US-A1- 2011 200 568
- US-A1- 2011 250 182
- US-A1- 2011 260 727
- SINGLA D K ET AL: "Induced Pluripotent Stem (iPS) Cells Repair and Regenerate Infarcted Myocardium", MOLECULAR PHARMACEUTICS, vol. 8, no. 5, 3 October 2011 (2011-10-03) , pages 1573-1581, XP002723017, ISSN: 1543-8384, DOI: 10.1021/MP2001704 [retrieved on 2011-05-24]
- SINGLA D K ET AL: "Enhancement by growth factors of cardiac myocyte differentiation from embryonic stem cells: A promising foundation for cardiac regeneration", BIOCHEMICAL AND BIOPHYSICAL RESEARCH COMMUNICATIONS, vol. 335, no. 3, 30 September 2005 (2005-09-30), pages 637-642, XP027230121, ISSN: 0006-291X, DOI: 10.1016/J.BBRC.2005.06.105 [retrieved on 2005-08-20]
- SINGLA D K ET AL: "Factors released from embryonic stem cells inhibit apoptosis of H9c2 cells", AMERICAN JOURNAL OF PHYSIOLOGY: HEART AND CIRCULATORY PHYSIOLOGY, vol. 293, no. 3, June 2007 (2007-06), pages H1590-H1595, XP55112889, US ISSN: 0363-6135, DOI: 10.1152/ajpheart.00431.2007
- SINGLA.: 'Embryonic Stem Cells in Cardiac Repair and Regeneration.' ANTIOXIDANTS & REDOX SIGNALING vol. 11, no. 8, 2009, pages 1857 - 1863, XP055112853

## Description

Parts of this invention were made with government support under 5R01HL090646-04 awarded by the National Institutes of Heath. The United States government has certain rights in the invention.

### BACKGROUND

Myocardial infarction (MI) leads to heart failure implicated by complex mechanisms of cardiac myocyte cell death, hypertrophy, and fibrosis. The common therapeutic preferences to inhibit further deterioration of MI leading to end stage heart failure are very restricted. Heart transplantation required to manage end stage heart failure patients is not readily available. Therefore, new cellular therapies to repair and regenerate injured myocardium as well as to prevent the progression of adverse cardiac remodeling are under diligent examination.

Thus far, cell transplantation in the mouse, rat, and human infarcted hearts has been studied employing a wide variety of cell types such as skeletal myoblasts, embryonic stem (ES) cells, c-kit positive cardiac stem cells, CD45^{+ve} bone marrow stem cells, mesenchymal stem cells (MSC), and hematopoietic stem cells. Indeed, adult stem cells in clinical trials have furnished significant insights into cell transplantation; however, their effectiveness is still in question. In contrast, ES cells have never reached clinical trials due to ethical and additional concerns. Thus, the optimal cell types for cardiac regeneration, repair and remodeling for transplantation in the injured myocardium has yet to be identified.

Despite advances in understanding the physiology and pathophysiology of cardiac dysfunction, including myocardial infarction, there is still a scarcity of compounds that are both potent, efficacious, and safe in the treatment of cardiac dysfunction. These needs and other needs are satisfied by the present invention.

Singla et al., Molecular Pharmaceutics 8 (5), 1573-1581 (2011) report on the reprogramming of H9c2 cells into iPS cells with the factors Oct3/4, Sox2, Klf4, and c-Myc. Singla et al., Am. J. Phys. Heart Circ. Physiol. 293(3) H1590-H1595 (2007) report that factors released from embryonic stem (ES) cells inhibit apoptosis of H9c2 cells. Antiapoptotic effects of ES cell conditioned media were inhibited with a TIMP-1 antibody. US patent application US 2002/142457 A1 discloses inter alia methods for proliferating cells having the potential to differentiate into cardiomyocytes and for regulating their differentiation into cardiomyocytes using various cytokines and transcription factors. US patent application US 2011/097799 A1 discloses inter alia methods for differentiation of stem cells into cardiomyocytes that overcome variability between different stem cell clones and different batch of culture medium. US patent application US 2002/061837 A1 discloses a composition comprising a purified mixture of a bone morphogenetic protein (BMP) and a fibroblast growth factor (FGF). International patent application WO 2006/081190 A2 discloses pharmaceutical polypeptide compositions, which may include one or more members of the FGF family. European patent application EP 2 098 244 A1 relates to the use of inter alia FGF9 for use in the treatment of heart diseases. International patent application WO 2009/118928 A1 discloses a method for producing cardiomyocytes and/or cardiac progenitor cells, in which an induced pluripotent stem (iPS) cell, which has been differentiated into a mesoderm cell, is cultured in the presence of cyclosporin-A.

### SUMMARY

The present invention relates to a composition comprising FGF-8 and/or FGF-8 and FGF-9 for use in a method of regenerative medicine according to independent claim 1.

Disclosed herein is furthermore a use in enhancing cardiac tissue and/or cell regeneration following cardiac dysfunction in a subject in need thereof, the use comprising (i) administering to the subject fibroblast growth factor-8 primed induced pluripotent stem cells; (ii) administering to the subject conditioned media of fibroblast growth factor-8 primed induced pluripotent stem cells; (iii) administering to the subject fibroblast growth factor-8; and/or (iv) administering to the subject a composition comprising fibroblast growth factor-8 and fibroblast growth factor-9, and determining regeneration in the heart.

Disclosed herein is also a use in attenuating adverse cardiac tissue and/or cell remodeling following cardiac dysfunction in a subject in need thereof, the use comprising (i) administering to the subject fibroblast growth factor-8 primed induced pluripotent stem cells; (ii) administering to the subject conditioned media of fibroblast growth factor-8 primed induced pluripotent stem cells; (iii) administering to the subject fibroblast growth factor-8; and/or (iv) administering to the subject a composition comprising fibroblast growth factor-8 and fibroblast growth factor-9, and determining a decrease in cardiac remodeling.

Disclosed herein is a composition for use in enhancing cardiac tissue/cell regeneration in a subject, comprising (i) fibroblast growth factor-8 primed induced pluripotent stem cells; (ii) conditioned medium of fibroblast growth factor-8 primed induced pluripotent stem cells; (iii) fibroblast growth factor-8; and/or (iv) a composition comprising fibroblast growth factor-8 and fibroblast growth factor-9.

Disclosed herein is a composition for use in attenuating adverse cardiac tissue/cell remodeling in a subject, comprising (i) fibroblast growth factor-8 primed induced pluripotent stem cells; (ii) conditioned medium of fibroblast growth factor-8 primed induced pluripotent stem cells; (iii) fibroblast growth factor-8; and/or (iv) fibroblast growth factor-8 and fibroblast growth factor-9.

Disclosed herein is a method of and a use in generating cardiac induced pluripotent stem cells, the method/use comprising (i) inserting one or more nucleic acid constructs capable of expressing stem-cell like factors into a cardiac cell type, wherein the stem-cell like factors comprise Oct3/4, KIf4, Sox2, and c-Myc, or a combination thereof; and (ii) obtaining the cardiac induced pluripotent stem cells stably expressing the stem-cell like factors in the cardiac cell type.

Disclosed herein is a method of inhibiting apoptosis and/or apoptosis-related mechanisms, comprising administering conditioned medium from induced pluripotent stem cells, wherein the conditioned medium is administered with or without FGF-8.

Disclosed herein are a method of and a use in identifying a myocardial injury or infarcted myocardial tissue, comprising measuring the amount of miR-486, wherein the amount of miR-486 decreases after myocardial injury or myocardial infarction, and wherein the amount of miR-486 increases after transplantation of induced pluripotent stem cells.

Disclosed herein are a method of and a use in inhibiting apoptosis and/or apoptosis-related mechanisms, comprising administering FGF-8.

### BRIEF DESCRIPTION OF THE FIGURES

The accompanying figures, which are incorporated in and constitute a part of this specification, illustrate several aspects and together with the description serve to explain the principles of the invention.
Figure 1 shows indicia of stably transfected iPS cells.
Figure 2 shows that iPS cells generated from embryoid bodies differentiate into cardiac myocytes, smooth muscles ells, and endothelial cells following FGF-8 treatment.
Figure 3 shows the effects of FGF-8 on beating cardiac myocytes derived from the iPS-EB cell system.
Figure 4 shows the effects of both iPS-CM and FGF-8 on H9c2 cells following H₂O₂-induced apoptosis.
Figure 5 shows that transplanted RFP-iPS or ES cells differentiate into cardiac myocytes with electrical coupling.
Figure 6 shows that transplanted iPS cells inhibit apoptosis at 2 weeks post-MI.
Figure 7 shows MMP-2 expression in ES and iPS cell transplanted hearts.
Figure 8 shows reduced fibrosis and improved cardiac function following iPS cell transplantation.
Figure 9 shows the effect of FGF-8 treatment on cardiac myocyte apoptosis following MI and the effect of FGF-8 treatment on Bax and Bcl-2 expression.
Figure 10 shows a decrease in levels of miR-486 following MI and the effect of iPS cell transplantation.
Figure 11 shows the effect of treatment with ES and iPS cells following MI on p-Akt and PTEN expression as well as the effect of FGF-8 treatment on p-Akt and PTEN expression.

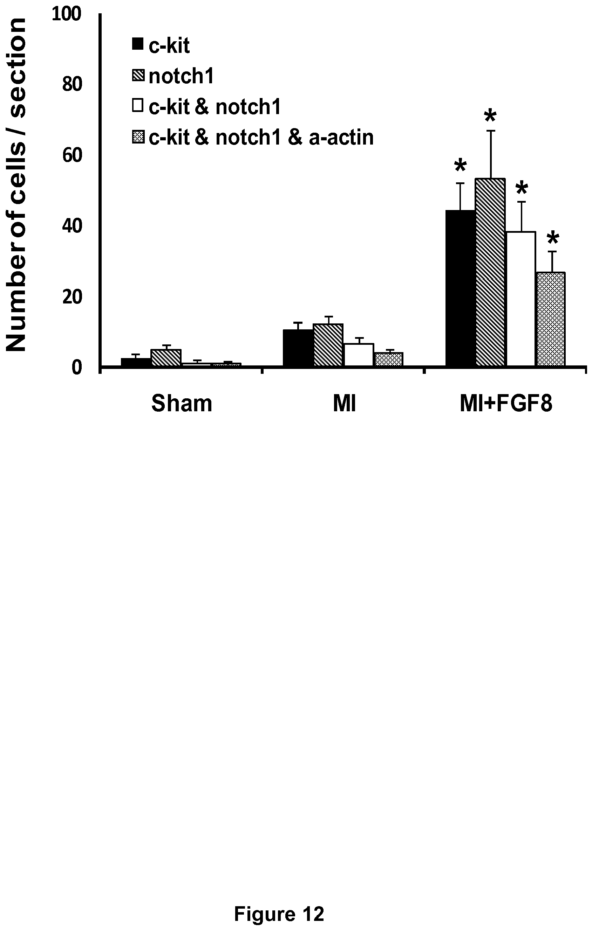

Figure 12 shows expression data of c-kit and notch1 following sham treatment and myocardial infarction both with and without FGF-8 treatment.
Figure 13 shows histological and quantifiable data regarding the effect of sham-MI, MI, and MI+FGF-8 treatment on interstitial fibrosis and vascular fibrosis / vascular area (VF/VA).
Figure 14 shows various cardiac performance measures following sham-MI, MI, and MI with FGF-8 treatment.

Additional advantages of the invention will be set forth in part in the description which follows, and in part will be obvious from the description, or can be learned by practice of the invention. The advantages of the invention will be realized and attained by means of the elements and combinations particularly pointed out in the appended claims.

### DETAILED DESCRIPTION

Induced pluripotent stem (iPS) cells could be a potential cell type of choice to treat heart diseases but their basic mechanisms of cardiac repair and regeneration are largely unknown. Using four (4) factors (Oct3/4, Sox2, Klf4, and c-Myc), iPS cells were generated from H9c2 cells (originally isolated from embryonic rat heart ventricular tissue). These cells have the potential to differentiate into cardiac myocytes in vitro and in vivo. Following myocardial infarction, transplanted cardiac iPS cells inhibited post-MI remodeling and demonstrated cardiac regeneration.

Data indicate that FGF family members play an important role in various cell signal transduction pathways associated with embryo development (Dyer et al., 2009; Yun et al., 2010). Specifically, FGF-8 has a unique role in cell differentiation, proliferation, and specification. (Dyer et al., 2009). FGF-8 mutant mice demonstrate abnormalities in the heart, suggesting its role as a key growth factor required for cardiac development (Dyer et al., 2009). The role of FGF-8 in the injured adult heart or iPS cells is previously unexplored. The data presented herein demonstrate that FGF-8 contains both pro-cardiac and anti-apoptotic properties and that FGF-8 inhibits cardiac myocyte apoptosis in vitro and in vivo.

### A. COMPOSITIONS

Disclosed herein are compositions useful in a method of enhancing cardiac tissue and/or cell regeneration. Disclosed herein are compositions useful in a method of attenuating cardiac tissue and/or cell remodeling. Disclosed herein are compositions useful in a method of inhibiting apoptosis and/or apoptosis-related mechanisms.

### 1. COMPOSITIONS FOR ENHANCEMENT OF CARDIAC TISSUE

### AND/OR CELL REGENERATION

Compositions disclosed herein enhance cardiac tissue/cell regeneration. Disclosed herein is a composition for enhancing cardiac tissue/cell regeneration in a subject, comprising: (A) fibroblast growth factor-8 primed induced pluripotent stem cells; (B) conditioned medium of fibroblast growth factor-8 primed induced pluripotent stem cells; (C) fibroblast growth factor-8; and/or (D) a composition comprising fibroblast growth factor-8 and fibroblast growth factor-9. For example, a disclosed composition for enhancing cardiac tissue and/or cell regeneration in a subject comprises any one of the following combinations of fibroblast growth factor-8 primed induced pluripotent stem cells (FGF-8 primed iPS cells); conditioned medium of fibroblast growth factor-8 primed induced pluripotent stem cells (CM of FGF-8 primed iPS cells); fibroblast growth factor-8 (FGF-8); and a composition comprising fibroblast growth factor-8 and fibroblast growth factor-9 (FGF-8 and FGF-9).

**Table 1 - Listing of Disclosed Compositions**

| Listing of Various Components of Disclosed Compositions | | | |
|---|---|---|---|
| Comprising A (i.e., FGF-8 primed | Comprising B (i.e., CM of FGF-8 iPS cells) | Comprising C (i.e., FGF-8) | Comprising D (i.e., FGF-8 & FGF-9) |
| A | B | C | D |
| A+B | B+A | C+A | D+A |
| A+C | B+C | C+B | D+B |
| A+D | B+D | C+D | D+C |
| A+B+C | B+A+C | C+A+B | D+A+B |
| A+B+D | B+A+D | C+A+D | D+A+C |
| A+C+D | B+C+D | C+B+D | D+B+C |
| A+B+C+D | B+A+C+D | C+A+B+D | D+A+B+C |

Thus, as detailed in the above table, a disclosed composition comprises one or two or three or all four of the following: (A) fibroblast growth factor-8 primed induced pluripotent stem cells; (B) conditioned media of fibroblast growth factor-8 primed induced pluripotent stem cells; (C) fibroblast growth factor-8; and (D) a composition comprising fibroblast growth factor-8 and fibroblast growth factor-9.

In an aspect, a disclosed composition protects myocardium including, but not limited to human myocardium, when administered with or without iPS cells.

In an aspect, a disclosed composition for enhancing cardiac tissue/cell regeneration in a subject enhances cells engraftment. In an aspect, a disclosed composition enhances cell proliferation. In an aspect, a disclosed composition enhances cell differentiation. In an aspect, a disclosed composition can perform any combination of the following: enhancing cell engraftment, enhancing cell proliferation, and/or enhancing cell differentiation.

In an aspect, the induced pluripotent stem cells differentiate into cardiac myocytes. In a further aspect, the induced pluripotent stem cells are cardiac-committed. In an aspect, a disclosed composition enhances cardiac myocyte differentiation in iPS cells or any other source of stem cells in regenerative medicine including, but not limited to, mesenchymal stem cells, adipocyte stems cells, and any other types of stem cells known to be effective by those of skill in the art. In an aspect, a disclosed composition enhances c-kit positive cells or any other endogenous heart stem cells.

In an aspect, a disclosed composition for enhancing cardiac tissue/cell regeneration in a subject improves angiogenesis in the subject. In an aspect, a disclosed composition enhances cardiac function in the subject. In an aspect, a disclosed composition both improves angiogenesis and enhances cardiac function in the subject.

In an aspect, enhancing cardiac function in a disclosed use comprises one or more of (i) improving left ventricular function, (ii) improving fractional shortening, (iii) improving ejection fraction, (iv) reducing end-diastolic volume, (v) decreasing left ventricular mass, and (vi) normalizing of heart geometry. In an aspect, enhancing cardiac function in a disclosed use comprises all of the following: (i) improving left ventricular function, (ii) improving fractional shortening, (iii) improving ejection fraction, (iv) reducing end-diastolic volume, (v) decreasing left ventricular mass, and (vi) normalizing of heart geometry. In an aspect, enhancing cardiac function in a disclosed use comprises any combination of (i) improving left ventricular function, (ii) improving fractional shortening, (iii) improving ejection fraction, (iv) reducing end-diastolic volume, (v) decreasing left ventricular mass, and/or (vi) normalizing of heart geometry.

In an aspect, the conditioned media of a disclosed composition comprises one or more anti-apoptotic and anti-fibrotic factors. In a further aspect, the one or more anti-apoptotic and anti-fibrotic factors comprise fibroblast growth factor-8 (FGF-8), fibroblast growth factor-9 (FGF-9), interleukin-10 (IL-10), and tissue inhibitor of matrix metalloproteinase-1 (TIMP-1).

In an aspect, the subject has experienced a cardiac ischemia/reperfusion event. In an aspect, the ischemia/reperfusion event is myocardial infarction, myocardial ischemia, myocardial reperfusion, subendocardial ischemia, Takayasu's arteritis, atrial fibrillation, hemorrhagic stroke, an event that occurs during cardiac surgery where a heart lung machine is used such as coronary artery bypass, or an event that occurs during the preservation of an organ for transplant. In an aspect, the subject has a congenital heart defect. In an aspect, the congenital heart defect is hypoplasia or pentalogy of Cantrell.

In an aspect, a disclosed composition for enhancing cardiac tissue/cell regeneration in a subject is administered to the subject prior to a cardiac ischemia/reperfusion event. In an aspect, a disclosed composition is administered to the subject during a cardiac ischemia/reperfusion event. In an aspect, a disclosed composition is administered to the subject following a cardiac ischemia/reperfusion event. For example, in an aspect, a disclosed composition is administered to the subject within 10, 15, 20, 25, 30, or more minutes following a cardiac ischemia/reperfusion event. In an aspect, a disclosed composition is administered within 1, 2, 6, 12, 18, 24, or more hour following a cardiac ischemia/reperfusion event.

In an aspect, a disclosed composition is administered to the subject one or more times. For example, in an aspect, a disclosed composition is administered to the subject prior to and during a cardiac ischemia/reperfusion event. In an aspect, a disclosed composition is administered to the subject during and following a cardiac ischemia/reperfusion event. For example, in an aspect, a disclosed composition is administered to the subject prior to and following a cardiac ischemia/reperfusion event. In an aspect, a disclosed composition is administered to the subject prior to, during, and following a cardiac ischemia/reperfusion event.

In an aspect, a disclosed composition for enhancing cardiac tissue/cell regeneration in a subject comprises between 5,000 and 500,000 induced pluripotent stem cells (iPS cells). In an aspect, a composition comprises approximately 100,000 iPS cells. In an aspect, a composition comprises less than 100,000 iPS cells. In an aspect, a composition comprises more than 100,000 iPS cells.

In an aspect, a disclosed composition for enhancing cardiac tissue/cell regeneration in a subject is administered to the subject in one intramyocardial injection. In an aspect, a disclosed composition is administered in two or more intramyocardial injections.

In an aspect, a disclosed composition for enhancing cardiac tissue/cell regeneration in a subject is administered into a peri-infarct zone of the injured myocardium. In an aspect, disclosed composition is administered into an infarcted zone of the injured myocardium. In an aspect, a disclosed composition is administered into a peri-infarct zone of the injured myocardium and into an infarcted zone of the injured myocardium.

In an aspect, a disclosed composition for enhancing cardiac tissue/cell regeneration in a subject further comprises one or more immunosuppressive drugs. Immunosuppressive drugs are known in the art. In an aspect, the one or more immunosuppressive drugs comprise corticosteroids, calcineurin inhibitors, anti-proliferatives, and mTOR inhibitors. In an aspect, the one or more immunosuppressive drugs can be a combination of immunosuppressive drugs. In an aspect, the one or more immunosuppressive drugs is cyclosporine A.

In an aspect, the induced pluripotent stem cells of a disclosed composition are obtained from an autologous source. In an aspect, the induced pluripotent stem cells are obtained from an allogeneic source. In an aspect, the induced pluripotent stem cells are obtained from a syngeneic source. In an aspect, the induced pluripotent stem cells are obtained from a combination of sources.

In yet another aspect, the induced pluripotent stem cells of a disclosed composition for enhancing cardiac tissue/cell regeneration in a subject are obtained from fibroblast cells. In a further aspect, the induced pluripotent stem cells are obtained from H9c2 cells. In an aspect, the fibroblast cells or the H9c2 cells are transfected with a vector comprising a nucleic acid molecule encoding at least one sternness factor. In an aspect, the at least one sternness factor comprises c-myc, oct 3/4, Klf4, nanog, or Sox2, or a combination thereof. For example, in an aspect, the sternness factors are c-myc, oct 3/4, Klf4, and Sox2.

In an aspect, a disclosed composition enhances neovascularization in iPS cells and/or any other stem cells used in regenerative medicine including, but not limited to mesenchymal stem cells, adipocyte stems cells, etc.

In an aspect, the subject is a mammal. In an aspect, the mammal is a primate. In an aspect, the mammal is a human. In an aspect, the human is a patient.

### 2. COMPOSITIONS FOR ATTENUATION OF ADVERSE CARDIAC TISSUE/CELL REMODELING

Compositions disclosed herein attenuate adverse cardiac tissue/cell remodeling. Disclosed herein is a composition for attenuating adverse cardiac tissue/cell remodeling in a subject, comprising: (A) fibroblast growth factor-8 primed induced pluripotent stem cells; (B) conditioned medium of fibroblast growth factor-8 primed induced pluripotent stem cells; (C) fibroblast growth factor-8; and/or (D) a composition comprising fibroblast growth factor-8 and fibroblast growth factor-9. For example, a disclosed composition for enhancing cardiac tissue and/or cell regeneration in a subject comprises any one of the following combinations of fibroblast growth factor-8 primed induced pluripotent stem cells (FGF-8 primed iPS cells); conditioned medium of fibroblast growth factor-8 primed induced pluripotent stem cells (CM of FGF-8 primed iPS cells); fibroblast growth factor-8 (FGF-8); and a composition comprising fibroblast growth factor-8 and fibroblast growth factor-9 (FGF-8 and FGF-9). For example, a disclosed composition for attenuating adverse cardiac tissue/cell remodeling in a subject comprises any one of the following:

**Table 2 - Listing of Disclosed Compositions**

| Listing of Various Components of Disclosed Compositions | | | |
|---|---|---|---|
| Comprising A (i.e., FGF-8 primed iPS cells) | Comprising B (i.e., CM of FGF-8 iPS cells) | Comprising C (i.e., FGF-8) | Comprising D (i.e., FGF-8 & FGF-9) |
| A | B | C | D |
| A+B | B+A | C+A | D+A |
| A+C | B+C | C+B | D+B |
| A+D | B+D | C+D | D+C |
| A+B+C | B+A+C | C+A+B | D+A+B |
| A+B+D | B+A+D | C+A+D | D+A+C |
| A+C+D | B+C+D | C+B+D | D+B+C |
| A+B+C+D | B+A+C+D | C+A+B+D | D+A+B+C |

Thus, as detailed in the above table, a disclosed composition comprises one or two or three or all four of the following: (A) fibroblast growth factor-8 primed induced pluripotent stem cells; (B) conditioned media of fibroblast growth factor-8 primed induced pluripotent stem cells; (C) fibroblast growth factor-8; and (D) a composition comprising fibroblast growth factor-8 and fibroblast growth factor-9.

In an aspect, a disclosed composition protects myocardium including, but not limited to human myocardium, when administered with or without iPS cells.

In an aspect, a disclosed composition for attenuating adverse cardiac tissue/cell remodeling in a subject improves angiogenesis in the subject. In an aspect, a disclosed composition enhances cardiac function in the subject. In an aspect, a disclosed composition improves angiogenesis and enhances cardiac function in the subject.

In an aspect, a disclosed composition for attenuating adverse cardiac remodeling inhibits fibrosis and/or fibrosis-related mechanisms. In an aspect, a disclosed composition decreases the expression level or activity level of at least one matrix metalloproteinase. In an aspect, the at least one matrix metalloproteinase is MMP-2. In an aspect, the at least one matrix metalloproteinase is MMP-9. In an aspect, the at least one matrix metalloproteinase comprises MMP-2 and MMP-9.

In an aspect, a disclosed composition for attenuating adverse cardiac tissue and/or cell remodeling in a subject inhibits or decreases necrosis. In an aspect, a disclosed composition inhibits or decreases apoptosis and/or apoptosis-related mechanisms. In an aspect, the apoptosis and/or apoptosis-related mechanisms occurs in the induced pluripotent stem cells. In an aspect, the apoptosis and/or apoptosis-related mechanism occurs in cardiac tissue of the subject. In an aspect, a disclosed composition increases miR-486 expression, increases Akt expression, decreases PTEN expression, or decreases FOXO1a expression. In an aspect, a disclosed composition increases miR-486 expression, increases Akt expression, decreases PTEN expression, and decreases FOXO1a expression. In an aspect, a disclosed composition performs any combination of the following: increases miR-486 expression, increases Akt expression, decreases PTEN expression, and/or decreases FOXO1a expression.

In an aspect, the induced pluripotent stem cells differentiate into cardiac myocytes. In a further aspect, the induced pluripotent stem cells are cardiac-committed. In an aspect, a disclosed composition enhances cardiac myocyte differentiation in iPS cells or any other source of stem cells in regenerative medicine including, but not limited to, mesenchymal stem cells, adipocyte stems cells, etc. In an aspect, a disclosed composition enhances c-kit positive cells or any other endogenous heart stem cells.

In an aspect, enhancing cardiac function in a disclosed use comprises one or more of (i) improving left ventricular function, (ii) improving fractional shortening, (iii) improving ejection fraction, (iv) reducing end-diastolic volume, (v) decreasing left ventricular mass, and (vi) normalizing of heart geometry. In an aspect, enhancing cardiac function in a disclosed use comprises each of the following: (i) improving left ventricular function, (ii) improving fractional shortening, (iii) improving ejection fraction, (iv) reducing end-diastolic volume, (v) decreasing left ventricular mass, and (vi) normalizing of heart geometry. In an aspect, enhancing cardiac function in a disclosed use comprises any combination of (i) improving left ventricular function, (ii) improving fractional shortening, (iii) improving ejection fraction, (iv) reducing end-diastolic volume, (v) decreasing left ventricular mass, and/or (vi) normalizing of heart geometry.

In an aspect, the conditioned media of a disclosed composition comprises one or more anti-apoptotic and anti-fibrotic factors. In a further aspect, the one or more anti-apoptotic and anti-fibrotic factors comprise fibroblast growth factor-8 (FGF-8), fibroblast growth factor-9 (FGF-9), interleukin-10 (IL-10), and tissue inhibitor of matrix metalloproteinase-1 (TIMP-1).

In an aspect, the subject has experienced a cardiac ischemia/reperfusion event. In an aspect, the ischemia/reperfusion event is myocardial infarction, myocardial ischemia, myocardial reperfusion, subendocardial ischemia, Takayasu's arteritis, atrial fibrillation, hemorrhagic stroke, an event that occurs during cardiac surgery where a heart lung machine is used such as coronary artery bypass, or an event that occurs during the preservation of an organ for transplant. In an aspect, the subject has a congenital heart defect. In an aspect, the congenital heart defect is hypoplasia or pentalogy of Cantrell.

In an aspect, a disclosed composition for attenuating adverse cardiac tissue/cell remodeling in a subject is administered to the subject prior to a cardiac ischemia/reperfusion event. In an aspect, a disclosed composition is administered to the subject during a cardiac ischemia/reperfusion event. In an aspect, a disclosed composition is administered to the subject following a cardiac ischemia/reperfusion event. For example, in an aspect, a disclosed composition is administered to the subject within 10, 15, 20, 25, 30, or more minutes following a cardiac ischemia/reperfusion event. In an aspect, a disclosed composition is administered within 1, 2, 6, 12, 18, 24, or more hour following a cardiac ischemia/reperfusion event.

In an aspect, a disclosed composition is administered to the subject one or more times. For example, in an aspect, a disclosed composition is administered to the subject prior to and during a cardiac ischemia/reperfusion event. In an aspect, a disclosed composition is administered to the subject during and following a cardiac ischemia/reperfusion event. For example, in an aspect, a disclosed composition is administered to the subject prior to and following a cardiac ischemia/reperfusion event. In an aspect, a disclosed composition is administered to the subject prior to, during, and/or following a cardiac ischemia/reperfusion event.

In an aspect, a disclosed composition for attenuating adverse cardiac tissue/cell remodeling in a subject comprises between 5,000 and 500,000 induced pluripotent stem cells (iPS cells). In an aspect, a disclosed composition comprises approximately 100,000 iPS cells. In an aspect, a disclosed comprises less than 100,000 iPS cells. In an aspect, a disclosed composition comprises more than 100,000 iPS cells.

In an aspect, a disclosed composition for attenuating adverse cardiac tissue/cell remodeling in a subject is administered to the subject in one intramycardial injection. In an aspect, a disclosed composition is administered in two or more intramycardial injections.

In an aspect, a disclosed composition for attenuating adverse cardiac tissue/cell remodeling in a subject is administered into a peri-infarct zone of the injured myocardium. In an aspect, a disclosed composition is administered into an infarcted zone of the injured myocardium. In an aspect, a disclosed composition is administered into a peri-infarct zone of the injured myocardium and into an infarcted zone of the injured myocardium.

In an aspect, a disclosed composition for attenuating adverse cardiac tissue/cell remodeling in a subject further comprises one or more immunosuppressive drugs. Immunosuppressive drugs are known in the art. In an aspect, the one or more immunosuppressive drugs comprise corticosteroids, calcineurin inhibitors, anti-proliferatives, and mTOR inhibitors. In an aspect, the one or more immunosuppressive drugs can be a combination of immunosuppressive drugs. In an aspect, the one or more immunosuppressive drugs is cyclosporine A.

In an aspect, the induced pluripotent stem cells of a disclosed composition for attenuating adverse cardiac tissue/cell remodeling in a subject are obtained from an autologous source. In an aspect, the induced pluripotent stem cells are obtained from an allogeneic source. In an aspect, the induced pluripotent stem cells are obtained from a syngeneic source. In an aspect, the induced pluripotent stem cells are obtained from a combination of sources.

In yet another aspect, the induced pluripotent stem cells of a disclosed composition for attenuating adverse cardiac tissue/cell remodeling in a subject are obtained from fibroblast cells. In a further aspect, the induced pluripotent stem cells are obtained from H9c2 cells. In an aspect, the fibroblast cells or the H9c2 cells are transfected with a vector comprising a nucleic acid molecule encoding at least one sternness factor. In an aspect, the at least one sternness factor comprises c-myc, oct 3/4, Klf4, nanog, or Sox2, or a combination thereof. For example, in an aspect, the sternness factors are c-myc, oct 3/4, Klf4, and Sox2.

In an aspect, a disclosed composition enhances neovascularization in iPS cells and/or any other stem cells used in regenerative medicine including, but not limited to, mesenchymal stem cells, adipocyte stems cells, etc.

In an aspect, the subject is a mammal. In an aspect, the mammal is a primate. In an aspect, the mammal is a human. In an aspect, the human is a patient.

### 3. PHARMACEUTICAL COMPOSITIONS

In an aspect, there is disclosed a pharmaceutical composition comprising a disclosed composition for enhancing cardiac tissue and/or cell regeneration. There is furthermore disclosed a pharmaceutical composition comprising a disclosed composition for attenuating adverse cardiac tissue and/or cell remodeling. That is, a pharmaceutical composition can be provided comprising a therapeutically effective amount of at least one disclosed compositions and a pharmaceutically acceptable carrier.

### B. USES COMPRISING A DISCLOSED COMPOSITION

### 1. ENHANCING CARDIAC TISSUE AND/OR CELL REGENERATION

The invention relates to a composition for use in enhancing cardiac tissue and/or cell regeneration in a subject. Disclosed herein is also a composition for use in enhancing cardiac tissue and/or cell regeneration following cardiac dysfunction in a subject in need thereof, the use comprising: (A) administering to the subject fibroblast growth factor-8 primed induced pluripotent stem cells; (B) administering to the subject conditioned media of fibroblast growth factor-8 primed induced pluripotent stem cells; (C) administering to the subject fibroblast growth factor-8; and/or (D) administering to the subject a composition comprising fibroblast growth factor-8 and fibroblast growth factor-9; and determining regeneration in the heart. For example, a disclosed use in enhancing cardiac tissue and/or cell regeneration in a subject comprises any one of the following combinations of fibroblast growth factor-8 primed induced pluripotent stem cells (FGF-8 primed iPS cells); conditioned medium of fibroblast growth factor-8 primed induced pluripotent stem cells (CM of FGF-8 primed iPS cells); fibroblast growth factor-8 (FGF-8); and a composition comprising fibroblast growth factor-8 and fibroblast growth factor-9 (FGF-8 and FGF-9).

**Table 3 - Listing of Various Components of Disclosed Compositions used in Disclosed Uses**

| Listing of Various Components of Disclosed Compositions employed in Disclosed Uses | | | |
|---|---|---|---|
| Comprising A (i.e., FGF-8 primed iPS cells) | Comprising B (i.e., CM of FGF-8 iPS cells) | Comprising C (i.e., FGF-8) | Comprising D (i.e., FGF-8 & FGF-9) |
| A | B | C | D |
| A+B | B+A | C+A | D+A |
| A+C | B+C | C+B | D+B |
| A+D | B+D | C+D | D+C |
| A+B+C | B+A+C | C+A+B | D+A+B |
| A+B+D | B+A+D | C+A+D | D+A+C |
| A+C+D | B+C+D | C+B+D | D+B+C |
| A+B+C+D | B+A+C+D | C+A+B+D | D+A+B+C |

Thus, as detailed in the above table, a disclosed composition comprises one or two or three or all four of the following: (A) fibroblast growth factor-8 primed induced pluripotent stem cells; (B) conditioned media of fibroblast growth factor-8 primed induced pluripotent stem cells; (C) fibroblast growth factor-8; and (D) a composition comprising fibroblast growth factor-8 and fibroblast growth factor-9.

In an aspect, a disclosed use protects myocardium including, but not limited to, human myocardium, with or without the administration of iPS cells.

In an aspect of a disclosed use in enhancing cardiac tissue and/or cell regeneration, fibroblast growth factor-8 (FGF-8) is administered to the subject prior to the administration of the induced pluripotent stem cells (iPS cells). In an aspect, FGF-8 is administered to the subject during the administration of the iPS cells. In an aspect, FGF-8 is administered to the subject following the administration of the iPS cells. In an aspect, FGF-8 is administered to the subject prior to and during the administration of the iPS cells. In an aspect, FGF-8 is administered to the subject prior to and following the administration of the iPS cells. In an aspect, FGF-8 is administered to the subject during and following the administration of the iPS cells.

In an aspect of a disclosed use in enhancing cardiac tissue and/or cell regeneration, a composition comprising fibroblast growth factor-8 (FGF-8) and fibroblast growth factor-9 (FGF-9) is administered to the subject prior to the administration of the induced pluripotent stem cells (iPS cells). In an aspect, a composition comprising FGF-8 and FGF-9 is administered to the subject during the administration of the iPS cells. In an aspect, a composition comprising FGF-8 and FGF-9 is administered to the subject following the administration of the iPS cells. In an aspect, a composition comprising FGF-8 and FGF-9 is administered to the subject prior to and during the administration of the iPS cells. In an aspect, a composition comprising FGF-8 and FGF-9 is administered to the subject prior to and following the administration of the iPS cells. In an aspect, a composition comprising FGF-8 and FGF-9 is administered to the subject during and following the administration of the iPS cells.

In an aspect of a disclosed use in enhancing cardiac tissue and/or cell regeneration, the conditioned media is administered to the subject prior to the administration of the induced pluripotent stem cells (iPS cells). In an aspect, the conditioned media is administered to the subject during the administration of the iPS cells. In an aspect, the conditioned media is administered to the subject following the administration of the iPS cells. In an aspect the conditioned media is administered to the subject prior to and during the administration of the iPS cells. In an aspect, the conditioned media is administered to the subject prior to and following the administration of the iPS cells. In an aspect, the conditioned media is administered to the subject during and following the administration of the iPS cells.

In an aspect of a disclosed use in enhancing cardiac tissue and/or cell regeneration, the administration of induced pluripotent stem cells to the subject is repeated. In an aspect, the administration of fibroblast growth factor-8 to the subject is repeated. In an aspect, the administration of a composition comprising fibroblast growth factor-8 and fibroblast growth factor-9 to the subject is repeated. In an aspect, the administration of the conditioned media to the subject is repeated.

In an aspect of a disclosed use in enhancing cardiac tissue and/or cell regeneration following cardiac dysfunction in a subject in need therefore, the induced pluripotent stem cells (iPS cells) are administered to the subject prior to, during, and/or following cardiac dysfunction. For example, in an aspect, the iPS cells are administered to the subject prior to cardiac dysfunction. In an aspect, the iPS cells are administered to the subject, during cardiac dysfunction. In an aspect, the iPS cells administered to the subject following cardiac dysfunction. For example, in an aspect, the iPS cells are administered to the subject prior to and during cardiac dysfunction. In an aspect, the iPS cells are administered to the subject during and following cardiac dysfunction. In an aspect, the iPS cells are administered to the subject prior to and following cardiac dysfunction. In an aspect, the iPS cells are administered to the subject prior to, during, and/or following cardiac dysfunction. In an aspect, the induced pluripotent stem cells are administered within 10, 15, 20, 25, 30, or more minutes following cardiac dysfunction. In an aspect, the induced pluripotent stem cells are administered within 1, 2, 6, 12, 18, 24, or more hours following cardiac dysfunction.

In an aspect of a disclosed use, enhancing cardiac tissue and/or cell regeneration comprises enhancing cell engraftment. In an aspect, enhancing cardiac tissue and/or cell regeneration comprises enhancing cell differentiation. In an aspect of a disclosed use, enhancing cardiac tissue and/or cell regeneration comprises both enhancing cell engraftment and enhancing cell differentiation. In an aspect, the induced pluripotent stem cells differentiate into cardiac myocytes. In a further aspect, the induced pluripotent stem cells are cardiac-committed. In an aspect, a disclosed use comprises enhancing cardiac myocyte differentiation in iPS cells or any other source of stem cells in regenerative medicine including, but not limited to, mesenchymal stem cells, adipocyte stems cells, etc. In an aspect, a disclosed use enhances c-kit positive cells or any other endogenous heart stem cells.

In an aspect, a disclosed use of enhancing cardiac tissue and/or cell regeneration following cardiac dysfunction in a subject in need thereof further comprises improving angiogenesis in the subject. In an aspect, a disclosed use further comprises enhancing cardiac function in the subject. In an aspect, a disclosed use further comprises improving both angiogenesis and enhancing cardiac function in the subject.

In an aspect, enhancing cardiac function in a disclosed use comprises one or more of (i) improving left ventricular function, (ii) improving fractional shortening, (iii) improving ejection fraction, (iv) reducing end-diastolic volume, (v) decreasing left ventricular mass, and (vi) normalizing of heart geometry. In an aspect, enhancing cardiac function in a disclosed use comprises all of the following: (i) improving left ventricular function, (ii) improving fractional shortening, (iii) improving ejection fraction, (iv) reducing end-diastolic volume, (v) decreasing left ventricular mass, and (vi) normalizing of heart geometry. In an aspect, enhancing cardiac function in a disclosed use comprises any combination of (i) improving left ventricular function, (ii) improving fractional shortening, (iii) improving ejection fraction, (iv) reducing end-diastolic volume, (v) decreasing left ventricular mass, and/or (vi) normalizing of heart geometry.

In an aspect, the conditioned media of a use disclosed herein comprises one or more anti-apoptotic and anti-fibrotic factors. In a further aspect, the one or more anti- apoptotic and anti-fibrotic factors comprise fibroblast growth factor-8 (FGF-8), fibroblast growth factor-9 (FGF-9), interleukin-10 (IL-10), and tissue inhibitor of matrix metalloproteinase-1 (TIMP-1).

In an aspect of a disclosed use in enhancing cardiac tissue and/or cell regeneration following cardiac dysfunction in a subject in need thereof, between 5,000 and 500,000 induced pluripotent stem cells (iPS cells) are administered to the subject. In an aspect, approximately 100,000 iPS cells are administered to the subject. In an aspect, less than 100,000 iPS cells are administered to the subject. In an aspect, more than 100,000 iPS cells are administered to the subject.

In an aspect of a disclosed use in enhancing cardiac tissue and/or cell regeneration following cardiac dysfunction in a subject in need thereof, the induced pluripotent stem cells can be administered in one intramycardial injection. In an aspect, the induced pluripotent stem cells are administered in two or more intramycardial injections.

In an aspect of a disclosed use in enhancing cardiac tissue and/or cell regeneration following cardiac dysfunction in a subject in need thereof, the induced pluripotent stem cells are administered into a peri-infarct zone of the injured myocardium. In an aspect, the induced pluripotent stem cells are administered into an infarcted zone of the injured myocardium. In an aspect, the induced pluripotent stem cells are administered into a peri-infarct zone of the injured myocardium and into an infarcted zone of the injured myocardium.

In an aspect, a disclosed use in enhancing cardiac tissue and/or cell regeneration following cardiac dysfunction in a subject in need thereof further comprises administering to the subject one or more immunosuppressive drugs. Immunosuppressive drugs are known in the art. In an aspect, the one or more immunosuppressive drugs comprise corticosteroids, calcineurin inhibitors, anti-proliferatives, and mTOR inhibitors. In an aspect, the one or more immunosuppressive drugs can be a combination of immunosuppressive drugs. In an aspect, the one or more immunosuppressive drugs is cyclosporine A. In an aspect, the one or more immunosuppressive drugs can be administered to the subject prior to, during, and/or following the administration of the induced pluripotent stem cells. In an aspect, the one or more immunosuppressive drugs can be administered to the subject prior to the administration of the induced pluripotent stem cells. In an aspect, the one or more immunosuppressive drugs can be administered to the subject during the administration of the induced pluripotent stem cells. In an aspect, the one or more immunosuppressive drugs can be administered to the subject following the administration of the induced pluripotent stem cells. In an aspect, the one or more immunosuppressive drugs can be administered to the subject prior to and during the administration of the induced pluripotent stem cells. In an aspect, the one or more immunosuppressive drugs can be administered to the subject during and following the administration of the induced pluripotent stem cells. In an aspect, the one or more immunosuppressive drugs can be administered to the subject prior to and following the administration of the induced pluripotent stem cells.

In an aspect, the cardiac dysfunction of a disclosed issue in a cardiac ischemia/reperfusion event. In an aspect, the ischemia/reperfusion event is myocardial infarction, myocardial ischemia, myocardial reperfusion, subendocardial ischemia, Takayasu's arteritis, atrial fibrillation, hemorrhagic stroke, an event that occurs during cardiac surgery where a heart lung machine is used such as coronary artery bypass, or an event that occurs during the preservation of an organ for transplant. In an aspect, the subject has a congenital heart defect. In an aspect, the congenital heart defect is hypoplasia or pentalogy of Cantrell.

In an aspect, the induced pluripotent stem cells of a disclosed use in enhancing cardiac tissue and/or cell regeneration following cardiac dysfunction in a subject in need thereof are obtained from an autologous source. In an aspect, the induced pluripotent stem cells are obtained from an allogeneic source. In an aspect, the induced pluripotent stem cells are obtained from a syngeneic source. In an aspect, the induced pluripotent stem cells are obtained from a combination of sources.

In yet another aspect, the induced pluripotent stem cells of a disclosed use in enhancing cardiac tissue and/or cell regeneration following cardiac dysfunction in a subject in need thereof are obtained from fibroblast cells. In a further aspect, the induced pluripotent stem cells are obtained from H9c2 cells. In an aspect, the fibroblast cells or the H9c2 cells are transfected with a vector comprising a nucleic acid molecule encoding at least one sternness factor. In an aspect, the at least one sternness factor comprises c-myc, oct 3/4, Klf4, nanog, or Sox2, or a combination thereof. For example, in an aspect, the sternness factors are c-myc, oct 3/4, Klf4, and Sox2.

In an aspect, a disclosed use enhances neovascularization in iPS cells and/or any other stem cells used in regenerative medicine including, but not limited to mesenchymal stem cells, adipocyte stems cells, and other types of stem cells known to be effective by those of skill in the art.

In an aspect of a disclosed use in enhancing cardiac tissue and/or cell regeneration following cardiac dysfunction in a subject in need thereof, the subject is a mammal. In an aspect, the mammal is a primate. In an aspect, the mammal is a human. In an aspect, the human is a patient.

### 2. ATTENUATING ADVERSE CARDIAC TISSUE AND/OR CELL REMODELING

In one aspect, the invention relates to a composition for use in attenuating adverse cardiac tissue and/or cell remodeling following cardiac dysfunction in a subject. Disclosed herein is a use in attenuating adverse cardiac tissue and/or cell remodeling following cardiac dysfunction in a subject in need thereof, the use comprising: (A) administering to the subject fibroblast growth factor-8 primed induced pluripotent stem cells; (B) administering to the subject conditioned media of fibroblast growth factor-8 primed induced pluripotent stem cells; (C) administering to the subject fibroblast growth factor-8; and/or (D) administering to the subject a composition comprising fibroblast growth factor-8 and fibroblast growth factor- 9; and determining a decrease in cardiac remodeling. For example, a disclosed use in enhancing cardiac tissue and/or cell regeneration following cardiac dysfunction in a subject in need thereof, comprises any one of the following:

**Table 4 - Listing of Various Components of Disclosed Compositions used in Disclosed Uses**

| Listing of Various Components of Disclosed Compositions employed in Disclosed Uses | | | |
|---|---|---|---|
| Comprising A (FGF-8 primed iPS cells) | Comprising B (CM of FGF-8 iPS | Comprising C (FGF-8) | Comprising D (FGF-8 & FGF-9) |
| A | B | C | D |
| A+B | B+A | C+A | D+A |
| A+C | B+C | C+B | D+B |
| A+D | B+D | C+D | D+C |
| A+B+C | B+A+C | C+A+B | D+A+B |
| A+B+D | B+A+D | C+A+D | D+A+C |
| A+C+D | B+C+D | C+B+D | D+B+C |
| A+B+C+D | B+A+C+D | C+A+B+D | D+A+B+C |

Thus, as detailed in the table, a disclosed use comprises administering one or two or three or more or all four of the following: (A) fibroblast growth factor-8 primed induced pluripotent stem cells; (B) conditioned media of fibroblast growth factor-8 primed induced pluripotent stem cells; (C) fibroblast growth factor-8; and (D) a composition comprising fibroblast growth factor-8 and fibroblast growth factor-9.

In an aspect, a disclosed use protects myocardium including, but not limited to human myocardium, with or without the administration of iPS cells.

In an aspect of a disclosed use in attenuating adverse cardiac tissue and/or cell remodeling, fibroblast growth factor-8 (FGF-8) is administered to the subject prior to the administration of the induced pluripotent stem cells (iPS cells). In an aspect, FGF-8 is administered to the subject during the administration of the iPS cells. In an aspect, FGF-8 is administered to the subject following the administration of the iPS cells. In an aspect, FGF-8 is administered to the subject prior to and during the administration of the iPS cells. In an aspect, FGF-8 is administered to the subject prior to and following the administration of the iPS cells. In an aspect, FGF-8 is administered to the subject during and following the administration of the iPS cells.

In an aspect of a disclosed use in attenuating adverse cardiac tissue and/or cell remodeling, a composition comprising fibroblast growth factor-8 and fibroblast growth factor-9 is administered to the subject prior to the administration of the induced pluripotent stem cells (iPS cells). In an aspect, a composition comprising fibroblast growth factor-8 and fibroblast growth factor-9 is administered to the subject during the administration of the iPS cells. In an aspect, a composition comprising fibroblast growth factor-8 and fibroblast growth factor-9 is administered to the subject following the administration of the iPS cells. In an aspect, a composition comprising fibroblast growth factor-8 and fibroblast growth factor-9 is administered to the subject prior to and during the administration of the iPS cells. In an aspect, a composition comprising fibroblast growth factor-8 and fibroblast growth factor-9 is administered to the subject prior to and following the administration of the iPS cells. In an aspect, a composition comprising fibroblast growth factor-8 and fibroblast growth factor-9 is administered to the subject during and following the administration of the iPS cells.

In an aspect of a disclosed use in attenuating adverse cardiac tissue and/or cell remodeling, the conditioned media is administered to the subject prior to the administration of the induced pluripotent stem cells (iPS cells). In an aspect, the conditioned media is administered to the subject during the administration of the iPS cells. In an aspect, the conditioned media is administered to the subject following the administration of the iPS cells. In an aspect the conditioned media is administered to the subject prior to and during the administration of the iPS cells. In an aspect, the conditioned media is administered to the subject prior to and following the administration of the iPS cells. In an aspect, the conditioned media is administered to the subject during and following the administration of the iPS cells.

In an aspect of a disclosed use in attenuating adverse cardiac tissue and/or cell remodeling, the administration of induced pluripotent stem cells to the subject is repeated. In an aspect, the administration of fibroblast growth factor-8 to the subject is repeated. In an aspect, the administration of a composition comprising fibroblast growth factor-8 and fibroblast growth factor-9 to the subject is repeated. In an aspect, the administration of the conditioned media to the subject is repeated.

In an aspect of a disclosed use in attenuating adverse cardiac tissue and/or cell remodeling following cardiac dysfunction in a subject in need thereof, the induced pluripotent stem cells are administered to the subject prior to, during, and/or following cardiac dysfunction. For example, in an aspect, the induced pluripotent stem cells are administered to the subject prior to cardiac dysfunction. For example, in an aspect, the induced pluripotent stem cells are administered to the subject, during cardiac dysfunction. For example, in an aspect, the induced pluripotent stem cells are administered to the subject or following cardiac dysfunction. For example, in an aspect, the induced pluripotent stem cells are administered to the subject prior to and during cardiac dysfunction. For example, in an aspect, the induced pluripotent stem cells are administered to the subject during and following cardiac dysfunction. For example, in an aspect, the induced pluripotent stem cells are administered to the subject prior to and following cardiac dysfunction. For example, in an aspect, the induced pluripotent stem cells are administered to the subject prior to, during, and/or following cardiac dysfunction. In an aspect, the induced pluripotent stem cells are administered within 10, 15, 20, 25, 30, or more minutes following cardiac dysfunction. In an aspect, the induced pluripotent stem cells are administered within 1, 2, 6, 12, 18, 24, or more hours following cardiac dysfunction.

In an aspect of a disclosed use, attenuating adverse cardiac remodeling comprises inhibiting fibrosis and/or fibrosis-related mechanisms. In an aspect, inhibiting fibrosis and/or fibrosis-related mechanisms comprises decreasing the expression level or activity level of at least one matrix metalloproteinase. In an aspect, the at least one matrix metalloproteinase is MMP-2. In an aspect, the at least one matrix metalloproteinase is MMP-9. In an aspect, the at least one matrix metalloproteinase comprises MMP-2 and MMP-9.

In an aspect, the induced pluripotent stem cells differentiate into cardiac myocytes. In a further aspect, the induced pluripotent stem cells are cardiac-committed. In an aspect, a disclosed use enhances cardiac myocyte differentiation in iPS cells or any other source of stem cells in regenerative medicine including, but not limited to, mesenchymal stem cells, adipocyte stems cells, etc. In an aspect, a disclosed use enhances c-kit positive cells or any other endogenous heart stem cells.

In an aspect, a disclosed use in attenuating adverse cardiac tissue and/or cell remodeling following cardiac dysfunction in a subject in need thereof further comprises improving angiogenesis in the subject. In an aspect, a disclosed use further comprises enhancing cardiac function in the subject. In an aspect, a disclosed use further comprises improving angiogenesis and enhancing cardiac function in the subject.

In an aspect, enhancing cardiac function in a disclosed use comprises one or more of (i) improving left ventricular function, (ii) improving fractional shortening, (iii) improving ejection fraction, (iv) reducing end-diastolic volume, (v) decreasing left ventricular mass, and (vi) normalizing of heart geometry. In an aspect, enhancing cardiac function in a disclosed use comprises all of the following: (i) improving left ventricular function, (ii) improving fractional shortening, (iii) improving ejection fraction, (iv) reducing end-diastolic volume, (v) decreasing left ventricular mass, and (vi) normalizing of heart geometry. In an aspect, enhancing cardiac function in a disclosed use comprises any combination of (i) improving left ventricular function, (ii) improving fractional shortening, (iii) improving ejection fraction, (iv) reducing end-diastolic volume, (v) decreasing left ventricular mass, and/or (vi) normalizing of heart geometry.

In an aspect, a disclosed use in attenuating adverse cardiac tissue and/or cell remodeling following cardiac dysfunction in a subject in need thereof further comprise inhibiting or decreasing necrosis.

In an aspect, a disclosed use in attenuating adverse cardiac tissue and/or cell remodeling following cardiac dysfunction in a subject in need thereof further comprise inhibiting or decreasing apoptosis and/or apoptosis-related mechanisms. In an aspect, the apoptosis and/or apoptosis-related mechanism occurs in the induced pluripotent stem cells. In an aspect, the apoptosis and/or apoptosis-related mechanism occurs in cardiac tissue of the subject. In an aspect, the inhibiting or decreasing of apoptosis and/or apoptosis-related mechanism is accompanied by an increase in miR-486 expression, an increase in Akt expression, a decrease in PTEN expression, and/or a decrease in FOXO1a expression. In an aspect, the inhibiting or decreasing of apoptosis and/or apoptosis-related mechanism is accompanied by an increase in miR-486 expression, an increase in Akt expression, a decrease in PTEN expression, and a decrease in FOXO1a expression. In an aspect, the inhibiting or decreasing of apoptosis and/or apoptosis-related mechanism is accompanied by any combination of the following: an increase in miR-486 expression, an increase in Akt expression, a decrease in PTEN expression, and/or a decrease in FOXO1a expression.

In an aspect, the conditioned media of a method disclosed herein comprises one or more anti-apoptotic and anti-fibrotic factors. In a further aspect, the one or more anti- apoptotic and anti-fibrotic factors comprise fibroblast growth factor-8 (FGF-8), fibroblast growth factor-9 (FGF-9), interleukin-10 (IL-10), and tissue inhibitor of matrix metalloproteinase-1 (TIMP-1).

In an aspect of a disclosed use in attenuating adverse cardiac tissue and/or cell remodeling following cardiac dysfunction in a subject in need thereof, between 5,000 and 500,000 induced pluripotent stem cells (iPS cells) are administered to the subject. In an aspect, approximately 100,000 iPS cells are administered to the subject. In an aspect, less than 100,000 iPS cells are administered to the subject. In an aspect, more than 100,000 iPS cells are administered to the subject.

In an aspect of a disclosed use in attenuating adverse cardiac tissue and/or cell remodeling following cardiac dysfunction in a subject in need thereof, the induced pluripotent stem cells can be administered in one intramycardial injection. In an aspect, the induced pluripotent stem cells are administered in two or more intramycardial injections.

In an aspect of a disclosed use in attenuating adverse cardiac tissue and/or cell remodeling following cardiac dysfunction in a subject in need thereof, the induced pluripotent stem cells are administered into a peri-infarct zone of the injured myocardium. In an aspect, the induced pluripotent stem cells are administered into an infarcted zone of the injured myocardium. In an aspect of a discloseduse, the induced pluripotent stem cells are administered into a peri-infarct zone of the injured myocardium and into an infarcted zone of the injured myocardium.

In an aspect, a disclosed use in attenuating adverse cardiac tissue and/or cell remodeling following cardiac dysfunction in a subject in need thereof further comprises administering to the subject one or more immunosuppressive drugs. In an aspect, the one or more immunosuppressive drugs comprise corticosteroids, calcineurin inhibitors, anti- proliferatives, and mTOR inhibitors. In an aspect, the one or more immunosuppressive drugs can be a combination of immunosuppressive drugs. In an aspect, the one or more immunosuppressive drugs is cyclosporine A. In an aspect, the one or more immunosuppressive drugs can be administered to the subject prior to, during, and/or following the administration of the induced pluripotent stem cells. For example, in an aspect, the one or more immunosuppressive drugs can be administered to the subject prior to the administration of the induced pluripotent stem cells. In an aspect, the one or more immunosuppressive drugs can be administered to the subject during the administration of the induced pluripotent stem cells. In an aspect, the one or more immunosuppressive drugs can be administered to the subject following the administration of the induced pluripotent stem cells. In an aspect, the one or more immunosuppressive drugs can be administered to the subject prior to and during the administration of the induced pluripotent stem cells. In an aspect, the one or more immunosuppressive drugs can be administered to the subject during and following the administration of the induced pluripotent stem cells. In an aspect, the one or more immunosuppressive drugs can be administered to the subject prior to and following the administration of the induced pluripotent stem cells.

In an aspect, the cardiac dysfunction of a disclosed use in attenuating adverse cardiac tissue and/or cell remodeling following cardiac dysfunction in a subject in need thereof is a cardiac ischemia/reperfusion event. In an aspect, the ischemia/reperfusion event is myocardial infarction, myocardial ischemia, myocardial reperfusion, subendocardial ischemia, Takayasu's arteritis, atrial fibrillation, hemorrhagic stroke, an event that occurs during cardiac surgery where a heart lung machine is used such as coronary artery bypass, or an event that occurs during the preservation of an organ for transplant. In an aspect, the subject has a congenital heart defect. In an aspect, the congenital heart defect is hypoplasia or pentalogy of Cantrell.

In an aspect, the induced pluripotent stem cells of a disclosed use in attenuating adverse cardiac tissue and/or cell remodeling following cardiac dysfunction in a subject in need thereof are obtained from an autologous source. In an aspect, the induced pluripotent stem cells are obtained from an allogeneic source. In an aspect, the induced pluripotent stem cells are obtained from a syngeneic source. In an aspect, the induced pluripotent stem cells are obtained from a combination of sources.

In yet another aspect, the induced pluripotent stem cells of a disclosed use in attenuating adverse cardiac tissue and/or cell remodeling following cardiac dysfunction in a subject in need thereof are obtained from fibroblast cells. In a further aspect, the induced pluripotent stem cells are obtained from H9c2 cells. In an aspect, the fibroblast cells or the H9c2 cells are transfected with a vector comprising a nucleic acid molecule encoding at least one sternness factor. In an aspect, the at least one sternness factor comprises c-myc, oct 3/4, Klf4, nanog, or Sox2, or a combination thereof. For example, in an aspect, the sternness factors are c-myc, oct 3/4, Klf4, and Sox2.

In an aspect, a disclosed use enhances neovascularization in iPS cells and/or any other stem cells used in regenerative medicine including, but not limited to mesenchymal stem cells, adipocyte stems cells, etc.

In an aspect of a disclosed use enhancing cardiac tissue and/or cell regeneration following cardiac dysfunction in a subject in need thereof, the subject is a mammal. In an aspect, the mammal is a primate. In an aspect, the mammal is a human. In an aspect, the human is a patient.

### 3. GENERATING INDUCED PLURIPOTENT STEM CELLS

Disclosed herein is a method of generating cardiac induced pluripotent stem cells. In an aspect, a method of generating cardiac induced pluripotent stem cells comprises (i) inserting one or more nucleic acid constructs capable of expressing stem-cell like factors into a cardiac cell type, and (ii) obtaining the cardiac induced pluripotent stem cells stably expressing the stem-cell like factors in the cardiac cell type. In an aspect, the stem-cell like factors comprise Oct3/4, KIf4, Sox2, and c-Myc, or a combination thereof. In an aspect, the induced pluripotent stem cells differentiate into cardiac myocytes. In a further aspect, the induced pluripotent stem cells are cardiac-committed. In an aspect, a disclosed method enhances cardiac myocyte differentiation in iPS cells or any other source of stem cells in regenerative medicine including, but not limited to, mesenchymal stem cells, adipocyte stems cells, etc. In an aspect, a disclosed method enhances c-kit positive cells or any other endogenous heart stem cells.

In an aspect, the induced pluripotent stem cells are obtained from an autologous source. In an aspect, the induced pluripotent stem cells are obtained from an allogeneic source. In an aspect, the induced pluripotent stem cells are obtained from a syngeneic source. In yet another aspect, the induced pluripotent stem cells are obtained from fibroblast cells. In an aspect, the induced pluripotent stem cells are obtained from a combination of sources. In a further aspect, the induced pluripotent stem cells are obtained from H9c2 cells. In an aspect, the fibroblast cells or the H9c2 cells are transfected with a vector comprising a nucleic acid molecule encoding at least one sternness factor.

In an aspect, a disclosed induced pluripotent stem cells are used in attenuating adverse cardiac tissue and/or cell remodeling following cardiac dysfunction in a subject. In an aspect, a disclosed induced pluripotent stem cells are used enhancing cardiac tissue and/or cell regeneration in a subject. In an aspect, the subject is a
mammal. In an aspect, the mammal is a primate. In an aspect, the mammal is a human. In an aspect, the human is a patient.

In an aspect, a disclosed induced pluripotent stem cells are primed with fibroblast growth factor-8. In an aspect, the induced pluripotent stem cells are administered with one or more of the following: conditioned media of fibroblast growth factor-8 primed induced pluripotent stem cells, fibroblast growth factor-8, and a composition comprising fibroblast growth factor-8 and fibroblast growth factor-9.

In an aspect, the timing of the administration of the induced pluripotent stem cells varies. For example, in an aspect, the induced pluripotent stem cells are administered prior to administration of conditioned media of fibroblast growth factor-8 primed induced pluripotent stem cells, fibroblast growth factor-8, and/or a composition comprising fibroblast growth factor-8 and fibroblast growth factor-9. In an aspect, the induced pluripotent stem cells are administered during the administration of conditioned media of fibroblast growth factor-8 primed induced pluripotent stem cells, fibroblast growth factor-8, and/or a composition comprising fibroblast growth factor-8 and fibroblast growth factor-9. In an aspect, the induced pluripotent stem cells are administered following the administration of conditioned media of fibroblast growth factor-8 primed induced pluripotent stem cells, fibroblast growth factor-8, and/or a composition comprising fibroblast growth factor-8 and fibroblast growth factor-9. In an aspect, the administration of induced pluripotent stem cells to the subject is repeated. For example, in an aspect, the induced pluripotent stem cells are administered both prior to and during, or prior to and following, or during and following, the administration of conditioned media of fibroblast growth factor-8 primed induced pluripotent stem cells, fibroblast growth factor-8, and/or a composition comprising fibroblast growth factor-8 and fibroblast growth factor-9.

In an aspect, the induced pluripotent stem cells (iPS cells) are administered to the subject prior to, during, and/or following cardiac dysfunction. In an aspect, the iPS cells are administered to the subject prior to cardiac dysfunction. In an aspect, the iPS cells are administered to the subject, during cardiac dysfunction. In an aspect, the iPS cells are administered to the subject or following cardiac dysfunction. In an aspect, the iPS cells are administered to the subject prior to and during cardiac dysfunction. In an aspect, the iPS cells are administered to the subject during and following cardiac dysfunction. In an aspect, the iPS cells are administered to the subject prior to and following cardiac dysfunction. In an aspect, the iPS cells are administered to the subject prior to, during, and following cardiac dysfunction. In an aspect, the iPS cells are administered within 10, 15, 20, 25, 30, or more minutes following cardiac dysfunction. In an aspect, the iPS cells are administered within 1, 2, 6, 12, 18, 24, or more hours following cardiac dysfunction.

In an aspect, a disclosed induced pluripotent stem cells improve angiogenesis in a subject. In an aspect, a disclosed induced pluripotent stem cells enhance cardiac function in a subject. In an aspect, a disclosed induced pluripotent stem cells improve angiogenesis and enhance cardiac function in the subject. In an aspect, enhancing cardiac function comprises one or more of (i) improving left ventricular function, (ii) improving fractional shortening, (iii) improving ejection fraction, (iv) reducing end-diastolic volume, (v) decreasing left ventricular mass, and/or (vi) normalizing of heart geometry.

In an aspect, a disclosed induced pluripotent stem cells inhibit or decrease necrosis. In an aspect, a disclosed induced pluripotent stem cells inhibit or decrease apoptosis and/or apoptosis-related mechanisms.

In an aspect, between 5,000 and 500,000 induced pluripotent stem cells (iPS cells) are administered to a subject. In an aspect, approximately 100,000 iPS cells are administered to a subject. In an aspect, less than 100,000 iPS cells are administered to a subject. In an aspect, more than 100,000 iPS cells are administered to a subject.

In an aspect, the induced pluripotent stem cells are administered in one intramycardial injection. In an aspect, the induced pluripotent stem cells are administered in two or more intramycardial injections. In an aspect, the induced pluripotent stem cells are administered into a peri-infarct zone of the injured myocardium. In an aspect, the induced pluripotent stem cells are administered into an infarcted zone of the injured myocardium. In an aspect, the induced pluripotent stem cells are administered into a peri-infarct zone of the injured myocardium and into an infarcted zone of the injured myocardium.

In an aspect, a disclosed induced pluripotent stem cells are administered with one or more immunosuppressive drugs. In an aspect, the one or more immunosuppressive drugs comprise corticosteroids, calcineurin inhibitors, anti-proliferatives, and mTOR inhibitors. In an aspect, the one or more immunosuppressive drugs can be a combination of immunosuppressive drugs. In an aspect, the one or more immunosuppressive drugs is cyclosporine A. In an aspect, the one or more immunosuppressive drugs can be administered to the subject prior to, during, and/or following the administration of the induced pluripotent stem cells.

### 4. IDENTIFYING A MYOCARDIAL INJURY

Disclosed herein is a method of identifying a myocardial injury or infarcted myocardial tissue. In an aspect, a method of identifying a myocardial injury or infarcted myocardial tissue comprises measuring the amount of miR-486. In an aspect, the amount of miR-486 decreases after myocardial injury or myocardial infarction. In an aspect, wherein the amount of miR-486 increases after treatment of induced pluripotent stem cells. In an aspect, a disclosed method of identifying a myocardial injury or infracted myocardial tissue further comprises measuring the amount of Akt expression, PTEN expression, and/or FOXO1a expression.

In an aspect, a disclosed method of identifying a myocardial injury or infarcted myocardial tissue further comprises measuring apoptosis and/or apoptosis-related mechanisms. In an aspect, the inhibiting or decreasing of apoptosis and/or apoptosis-related mechanism is accompanied by an increase in miR-486 expression, an increase in Akt expression, a decrease in PTEN expression, and a decrease in FOXO1a expression. In an aspect, the inhibiting or decreasing of apoptosis and/or apoptosis-related mechanism is accompanied by any combination of the following: an increase in miR-486 expression, an increase in Akt expression, a decrease in PTEN expression, and a decrease in FOXO1a expression.

### 5. INHIBITING APOPTOSIS AND/OR APOPTOSIS-RELATED MECHANISMS

Disclosed herein are methods of and uses in inhibiting apoptosis and/or apoptosis-related mechanisms. In an aspect, a method of or a use in inhibiting apoptosis and/or apoptosis-related mechanisms comprises administering conditioned medium from induced pluripotent stem cells. In an aspect, a use in inhibiting apoptosis and/or apoptosis-related mechanisms comprises administering FGF-8. In an aspect, the conditioned media or the FGF-8 is administered to a subject.

In an aspect, the subject has experienced a cardiac ischemia/reperfusion event. In an aspect, the ischemia/reperfusion event is myocardial infarction, myocardial ischemia, myocardial reperfusion, subendocardial ischemia, Takayasu's arteritis, atrial fibrillation, hemorrhagic stroke, an event that occurs during cardiac surgery where a heart lung machine is used such as coronary artery bypass, or an event that occurs during the preservation of an organ for transplant. In an aspect, the subject has a congenital heart defect. In an aspect, the congenital heart defect is hypoplasia or pentalogy of Cantrell.

In an aspect, the conditioned media of a disclosed method or use comprises one or more anti-apoptotic and anti-fibrotic factors. In a further aspect, the one or more anti-apoptotic and anti-fibrotic factors comprise fibroblast growth factor-9, fibroblast growth factor-8, interleukin-10, and tissue inhibitor of matrix metalloproteinase-1. In an aspect, the conditioned medium is administered with or without fibroblast growth factor-8 (FGF-8). In an aspect, the conditioned medium is administered with or without fibroblast growth factor-9 (FGF-9). In an aspect, the conditioned medium is administered with or without interleukin-10 (IL-10). In an aspect, the conditioned medium is administered with or without matrix metalloproteinase-1 (MMP-1). In an aspect, the conditioned medium is administered with one or more of the following: fibroblast growth factor-9, fibroblast growth factor-8, interleukin-10, and tissue inhibitor of matrix metalloproteinase-1. In an aspect, the conditioned medium is administered with any combination of the following: fibroblast growth factor-9, fibroblast growth factor-8, interleukin-10, and tissue inhibitor of matrix metalloproteinase-1, such as, for example, the combination of FGF-8 and FGF-9.

In an aspect, a disclosed use protects myocardium including, but not limited to human myocardium, with or without the administration of iPS cells.

In an aspect, a disclosed use in inhibiting apoptosis and/or apoptosis- related mechanisms further comprises administering induced pluripotent stem cells. In an aspect, the induced pluripotent stem cells differentiate into cardiac myocytes. In a further aspect, the induced pluripotent stem cells are cardiac-committed. In an aspect, a disclosed use enhances cardiac myocyte differentiation in iPS cells or any other source of stem cells in regenerative medicine including, but not limited to, mesenchymal stem cells, adipocyte stems cells, etc. In an aspect, a disclosed use enhances c-kit positive cells or any other endogenous heart stem cells. In an aspect, the conditioned medium is administered to the subject prior to the administration of the induced pluripotent stem cells. In an aspect, the conditioned medium is administered during the administration of the induced pluripotent stem cells. In an aspect, the conditioned medium is administered following the administration of the induced pluripotent stem cells. In an aspect, the conditioned medium is administered the conditioned medium prior to and during the administration of the induced pluripotent stem cells. In an aspect, the conditioned medium is administered the conditioned medium prior to and following the administration of the induced pluripotent stem cells. In an aspect, the conditioned medium is administered to the subject during and following the administration of the induced pluripotent stem cells.

In an aspect, the apoptosis and/or apoptosis-related mechanisms occurs in the induced pluripotent stem cells. In an aspect, the apoptosis and/or apoptosis-related mechanisms occurs in cardiac tissue of the subject. In an aspect, a disclosed use increases miR-486 expression, increases Akt expression, decreases PTEN expression, and/or decreases FOXO1a expression. In an aspect, a disclosed composition increases miR-486 expression, increases Akt expression, decreases PTEN expression, and decreases FOXO1a expression. In an aspect, a disclosed composition performs any combination of the following: increases miR-486 expression, increases Akt expression, decreases PTEN expression, and decreases FOXO1a expression.

In an aspect, a disclosed use enhances neovascularization in iPS cells and/or any other stem cells used in regenerative medicine including, but not limited to mesenchymal stem cells, adipocyte stems cells, etc.

### 6. NON-MEDICAL USES

Also disclosed herein are uses of a disclosed composition as investigational and/or research tools in the development and standardization of *in vitro* and *in vivo* test systems for evaluation in laboratory animals such as cats, dogs, rabbits, monkeys, rats and mice, as part of the search for new therapeutic approaches for the enhancement of cardiac tissue and/or cell regeneration as well as the evaluation of the attenuation of adverse cardiac tissue and/or cell remodeling. In an aspect, these approaches apply to subjects with cardiac dysfunction.

### 7. DEFINITIONS

Unless otherwise expressly stated, it is in no way intended that any method or aspect set forth herein be construed as requiring that its steps be performed in a specific order. Accordingly, where a method claim does not specifically state in the claims or descriptions that the steps are to be limited to a specific order, it is no way intended that an order be inferred, in any respect. This holds for any possible non-express basis for interpretation, including matters of logic with respect to arrangement of steps or operational flow, plain meaning derived from grammatical organization or punctuation, or the number or type of aspects described in the specification.

As used in the specification and the appended claims, the singular forms "a," "an" and "the" include plural referents unless the context clearly dictates otherwise.

The word "or" as used herein means any one member of a particular list and also includes any combination of members of that list.

Ranges can be expressed herein as from "about" one particular value, and/or to "about" another particular value. When such a range is expressed, a further aspect includes from the one particular value and/or to the other particular value. Similarly, when values are expressed as approximations, by use of the antecedent "about," it will be understood that the particular value forms a further aspect. It will be further understood that the endpoints of each of the ranges are significant both in relation to the other endpoint, and independently of the other endpoint. It is also understood that there are a number of values disclosed herein, and that each value is also herein disclosed as "about" that particular value in addition to the value itself. For example, if the value "10" is disclosed, then "about 10" is also disclosed. It is also understood that each unit between two particular units are also disclosed. For example, if 10 and 15 are disclosed, then 11, 12, 13, and 14 are also disclosed.

As used herein, the amino acid abbreviations are conventional one letter codes for the amino acids and are expressed as follows: A, alanine; B, asparagine or aspartic acid; C, cysteine; D aspartic acid; E, glutamate, glutamic acid; F, phenylalanine; G, glycine; H histidine; I isoleucine; K, lysine; L, leucine; M, methionine; N, asparagine; P, proline; Q, glutamine; R, arginine; S, serine; T, threonine; V, valine; W, tryptophan; Y, tyrosine; Z, glutamine or glutamic acid.

"Peptide" as used herein refers to any peptide, oligopeptide, polypeptide, gene product, expression product, or protein. For example, a peptide can be an enzyme. A peptide is comprised of consecutive amino acids. The term "peptide" encompasses naturally occurring or synthetic molecules.

In general, the biological activity or biological action of a peptide refers to any function exhibited or performed by the peptide that is ascribed to the naturally occurring form of the peptide as measured or observed in vivo (i.e., in the natural physiological environment of the protein) or in vitro (i.e., under laboratory conditions). For example, a biological activity of caspase includes caspase enzymatic activity.

The term "enzyme" as used herein refers to any peptide that catalyzes a chemical reaction of other substances without itself being destroyed or altered upon completion of the reaction. Typically, a peptide having enzymatic activity catalyzes the formation of one or more products from one or more substrates. Such peptides can have any type of enzymatic activity including, without limitation, the enzymatic activity or enzymatic activities associated with enzymes such as those disclosed herein.

References in the specification and concluding claims to parts by weight of a particular element or component in a composition denotes the weight relationship between the element or component and any other elements or components in the composition or article for which a part by weight is expressed. Thus, in a compound containing 2 parts by weight of component X and 5 parts by weight component Y, X and Y are present at a weight ratio of 2:5, and are present in such ratio regardless of whether additional components are contained in the compound.

A weight percent (wt. %) of a component, unless specifically stated to the contrary, is based on the total weight of the formulation or composition in which the component is included.

As used herein, the terms "optional" or "optionally" means that the subsequently described event or circumstance can or can not occur, and that the description includes instances where said event or circumstance occurs and instances where it does not.

As used herein, the terms "transformation" and "transfection" mean the introduction of a nucleic acid, e.g., an expression vector, into a recipient cell including introduction of a nucleic acid to the chromosomal DNA of said cell. The art is familiar with various compositions, methods, techniques, etc. used to effect the introduction of a nucleic acid into a recipient cell. The art is familiar with such compositions, methods, techniques, etc for both eukaryotic and prokaryotic cells. The art is familiar with such compositions, methods, techniques, etc. for the optimization of the introduction and expression of a nucleic acid into and within a recipient cell.

As used herein, the term "subject" refers to the target of administration, e.g., an animal. Thus, the subject of the herein disclosed methods can be a vertebrate, such as a mammal, a fish, a bird, a reptile, or an amphibian. Alternatively, the subject of the herein disclosed methods can be a human, non-human primate, horse, pig, rabbit, dog, sheep, goat, cow, cat, guinea pig or rodent. The term does not denote a particular age or sex. Thus, adult and newborn subjects, as well as fetuses, whether male or female, are intended to be covered. In one aspect, the subject is a mammal. A patient refers to a subject afflicted with a disease or disorder. The term "patient" includes human and veterinary subjects. In some aspects of the disclosed methods, the subject has been diagnosed with a need for treatment for cardiac dysfunction, such as, for example, a cardiac ischemia/reperfusion event prior to the administering step.

As used herein, the term "treatment" refers to the medical management of a subject or a patient with the intent to cure, ameliorate, stabilize, or prevent a disease, pathological condition, or disorder, such as, for example, an injured myocardium. This term includes active treatment, that is, treatment directed specifically toward the improvement of a disease, pathological condition, or disorder, and also includes causal treatment, that is, treatment directed toward removal of the cause of the associated disease, pathological condition, or disorder. In addition, this term includes palliative treatment, that is, treatment designed for the relief of symptoms rather than the curing of the disease, pathological condition, or disorder; preventative treatment, that is, treatment directed to minimizing or partially or completely inhibiting the development of the associated disease, pathological condition, or disorder; and supportive treatment, that is, treatment employed to supplement another specific therapy directed toward the improvement of the associated disease, pathological condition, or disorder. In various aspects, the term covers any treatment of a subject, including a mammal (e.g., a human), and includes: (i) preventing the disease from occurring in a subject that can be predisposed to the disease but has not yet been diagnosed as having it; (ii) inhibiting the disease, i.e., arresting its development; or (iii) relieving the disease, i.e., causing regression of the disease. In an aspect, the disease, pathological condition, or disorder is cardiac dysfunction, such as, for example, a cardiac ischemia/reperfusion event.

As used herein, the term "prevent" or "preventing" refers to precluding, averting, obviating, forestalling, stopping, or hindering something from happening, especially by advance action. It is understood that where reduce, inhibit or prevent are used herein, unless specifically indicated otherwise, the use of the other two words is also expressly disclosed.

As used herein, the term "diagnosed" means having been subjected to a physical examination by a person of skill, for example, a physician, and found to have a condition that can be diagnosed or treated by compositions or methods disclosed herein. For example, "diagnosed with cardiac dysfunction" means having been subjected to a physical examination by a person of skill, for example, a physician, and found to have a condition that can be diagnosed or treated by a compound or composition that alleviates or ameliorates cardiac dysfunction. As a further example, "diagnosed with a need for improving or enhancing cardiac regeneration and/or cardiac function" refers to having been subjected to a physical examination by a person of skill, for example, a physician, and found to have a condition characterized by cardiac dysfunction and/or cardiac impairment and/or cardiac cell death, wherein improving or enhancing cardiac regeneration and/or cardiac function would be beneficial to the subject. Such a diagnosis can be in reference to a disorder, such as cardiac dysfunction, and the like, as discussed herein.

As used herein, the phrase "identified to be in need of treatment for a disorder," or the like, refers to selection of a subject based upon need for treatment of the disorder. For example, a subject can be identified as having a need for treatment of a disorder (e.g., a disorder related to cardiac dysfunction or myocardial infarction) based upon an earlier diagnosis by a person of skill and thereafter subjected to treatment for the disorder. It is contemplated that the identification can, in one aspect, be performed by a person different from the person making the diagnosis. It is also contemplated, in a further aspect, that the administration can be performed by one who subsequently performed the administration.

As used herein, the terms "administering" and "administration" refer to any method of providing a pharmaceutical preparation to a subject. Such methods are well known to those skilled in the art and include, but are not limited to, intracardiac administration, oral administration, transdermal administration, administration by inhalation, nasal administration, topical administration, intravaginal administration, ophthalmic administration, intraaural administration, intracerebral administration, rectal administration, sublingual administration, buccal administration, and parenteral administration, including injectable such as intravenous administration, intra-arterial administration, intramuscular administration, and subcutaneous administration. Administration can be continuous or intermittent. In various aspects, a preparation can be administered therapeutically; that is, administered to treat an existing disease or condition. In further various aspects, a preparation can be administered prophylactically; that is, administered for prevention of a disease or condition.

The term "contacting" as used herein refers to bringing a disclosed compound and a cell, target receptor, or other biological entity together in such a manner that the compound can affect the activity of the target (e.g., receptor, transcription factor, cell, etc.), either directly; i.e., by interacting with the target itself, or indirectly; i.e., by interacting with another molecule, co-factor, factor, or protein on which the activity of the target is dependent.

As used herein, the term "determining" can refer to measuring or ascertaining a quantity or an amount or a change in expression and/or activity level, e.g., of a nucleotide or transcript or polypeptide. For example, determining the amount of a disclosed transcript or polypeptide in a sample as used herein can refer to the steps that the skilled person would take to measure or ascertain some quantifiable value of the transcript or polypeptide in the sample. The art is familiar with the ways to measure an amount of the disclosed nucleotides, transcripts, polypeptides, etc.

As used herein, the term "level" refers to the amount of a target molecule in a sample, e.g., a sample from a subject. The amount of the molecule can be determined by any method known in the art and will depend in part on the nature of the molecule (i.e., gene, mRNA, cDNA, protein, enzyme, etc.). The art is familiar with quantification methods for nucleotides (e.g., genes, cDNA, mRNA, etc) as well as proteins, polypeptides, enzymes, etc. It is understood that the amount or level of a molecule in a sample need not be determined in absolute terms, but can be determined in relative terms (e.g., when compare to a control or a sham or an untreated sample).

As used herein, the terms "effective amount" and "amount effective" refer to an amount that is sufficient to achieve the desired result or to have an effect on an undesired condition. For example, a "therapeutically effective amount" refers to an amount that is sufficient to achieve the desired therapeutic result or to have an effect on undesired symptoms, but is generally insufficient to cause adverse side affects. The specific therapeutically effective dose level for any particular patient will depend upon a variety of factors including the disorder being treated and the severity of the disorder; the specific composition employed; the age, body weight, general health, sex and diet of the patient; the time of administration; the route of administration; the rate of excretion of the specific compound employed; the duration of the treatment; drugs used in combination or coincidental with the specific compound employed and like factors well known in the medical arts.

By "modulate" is meant to alter, by increase or decrease. As used herein, a "modulator" can mean a composition that can either increase or decrease the expression level or activity level of a gene or gene product such as a peptide. Modulation in expression or activity does not have to be complete. For example, expression or activity can be modulated by about 10%, 20%, 30%, 40%, 50%, 60%, 70%, 80%, 90%, 95%, 99%, 100% or any percentage in between as compared to a control cell wherein the expression or activity of a gene or gene product has not been modulated by a composition.

As used herein, "EC₅₀," is intended to refer to the concentration or dose of a substance (e.g., a compound or a drug) that is required for 50% enhancement or activation of a biological process, or component of a process, including a protein, subunit, organelle, ribonucleoprotein, etc. EC₅₀ also refers to the concentration or dose of a substance that is required for 50% enhancement or activation *in vivo*, as further defined elsewhere herein. Alternatively, EC₅₀ can refer to the concentration or dose of compound that provokes a response halfway between the baseline and maximum response. The response can be measured in a in vitro or in vivo system as is convenient and appropriate for the biological response of interest. For example, the response can be measured *in vitro* using cultured cardiac cells or in an *ex vivo* organ culture system with isolated cardiac cells, e.g., cardiomyocytes, vascular smooth muscle cells, endothelial cells, etc). Alternatively, the response can be measured *in vivo* using an appropriate research model such as rodent, including mice and rats. The mouse or rat can be an inbred strain with phenotypic characteristics of interest such as, for example, obesity or diabetes. As appropriate, the response can be measured in a transgenic or knockout mouse or rat wherein a gene or genes has been introduced or knocked-out, as appropriate, to replicate a disease process.

As used herein, "IC₅₀," is intended to refer to the concentration or dose of a substance (e.g., a compound or a drug) that is required for 50% inhibition or diminution of a biological process, or component of a process, including a protein, subunit, organelle, ribonucleoprotein, etc. IC₅₀ also refers to the concentration or dose of a substance that is required for 50% inhibition or diminution *in vivo*, as further defined elsewhere herein. Alternatively, IC₅₀ also refers to the half maximal (50%) inhibitory concentration (IC) or inhibitory dose of a substance. The response can be measured in a *in vitro* or *in vivo* system as is convenient and appropriate for the biological response of interest. For example, the response can be measured *in vitro* using cultured cardiac cells or in an *ex vivo* organ culture system with isolated cardiac cells (e.g., cardiomyocytes, vascular smooth muscle cells, endothelial cells, etc.). Alternatively, the response can be measured *in vivo* using an appropriate research model such as rodent, including mice and rats. The mouse or rat can be an inbred strain with phenotypic characteristics of interest such as, for example, obesity or diabetes. As appropriate, the response can be measured in a transgenic or knockout mouse or rat wherein a gene or genes has been introduced or knocked-out, as appropriate, to replicate a disease process.

The term "pharmaceutically acceptable" describes a material that is not biologically or otherwise undesirable, i.e., without causing an unacceptable level of undesirable biological effects or interacting in a deleterious manner. As used herein, the term "pharmaceutically acceptable carrier" refers to sterile aqueous or nonaqueous solutions, dispersions, suspensions or emulsions, as well as sterile powders for reconstitution into sterile injectable solutions or dispersions just prior to use. Examples of suitable aqueous and nonaqueous carriers, diluents, solvents or vehicles include water, ethanol, polyols (such as glycerol, propylene glycol, polyethylene glycol and the like), carboxymethylcellulose and suitable mixtures thereof, vegetable oils (such as olive oil) and injectable organic esters such as ethyl oleate. Proper fluidity can be maintained, for example, by the use of coating materials such as lecithin, by the maintenance of the required particle size in the case of dispersions and by the use of surfactants. These compositions can also contain adjuvants such as preservatives, wetting agents, emulsifying agents and dispersing agents. Prevention of the action of microorganisms can be ensured by the inclusion of various antibacterial and antifungal agents such as paraben, chlorobutanol, phenol, sorbic acid and the like. It can also be desirable to include isotonic agents such as sugars, sodium chloride and the like. Prolonged absorption of the injectable pharmaceutical form can be brought about by the inclusion of agents, such as aluminum monostearate and gelatin, which delay absorption. Injectable depot forms are made by forming microencapsule matrices of the drug in biodegradable polymers such as polylactide-polyglycolide, poly(orthoesters) and poly(anhydrides). Depending upon the ratio of drug to polymer and the nature of the particular polymer employed, the rate of drug release can be controlled. Depot injectable formulations are also prepared by entrapping the drug in liposomes or microemulsions which are compatible with body tissues. The injectable formulations can be sterilized, for example, by filtration through a bacterial-retaining filter or by incorporating sterilizing agents in the form of sterile solid compositions which can be dissolved or dispersed in sterile water or other sterile injectable media just prior to use. Suitable inert carriers can include sugars such as lactose. Desirably, at least 95% by weight of the particles of the active ingredient have an effective particle size in the range of 0.01 to 10 micrometers.

Disclosed are the components to be used to prepare a composition for use of the invention as well as the compositions themselves to be used within the methods disclosed herein. These and other materials are disclosed herein, and it is understood that when combinations, subsets, interactions, groups, etc. of these materials are disclosed that while specific reference of each various individual and collective combinations and permutation of these compounds can not be explicitly disclosed, each is specifically contemplated and described herein. For example, if a particular compound is disclosed and discussed and a number of modifications that can be made to a number of molecules including the compounds are discussed, specifically contemplated is each and every combination and permutation of the compound and the modifications that are possible unless specifically indicated to the contrary. Thus, if a class of molecules A, B, and C are disclosed as well as a class of molecules D, E, and F and an example of a combination molecule, A-D is disclosed, then even if each is not individually recited each is individually and collectively contemplated meaning combinations, A-E, A-F, B-D, B-E, B-F, C-D, C-E, and C-F are considered disclosed. Likewise, any subset or combination of these is also disclosed. Thus, for example, the subgroup of A-E, B-F, and C-E would be considered disclosed. This concept applies to all aspects of this application including, but not limited to, steps in methods of making and using the compositions that are employed in a use of the invention. Thus, if there are a variety of additional steps that can be performed it is understood that each of these additional steps can be performed with any specific embodiment or combination of embodiments of the uses of the invention.

The present invention can be understood more readily by reference to the following detailed description of the invention and the Examples included therein.

The publications discussed herein are provided solely for their disclosure prior to the filing date of the present application. Further, the dates of publication provided herein can be different from the actual publication dates, which can require independent confirmation.

### C. EXPERIMENTAL

The following examples are put forth so as to provide those of ordinary skill in the art with a complete disclosure and description of how the compounds, compositions, articles, devices and/or methods disclosed herein are made and evaluated.

Efforts have been made to ensure accuracy with respect to numbers (e.g., amounts, temperature, etc.), but some errors and deviations should be accounted for. Unless indicated otherwise, parts are parts by weight, temperature is in °C or is at ambient temperature, and pressure is at or near atmospheric.

### 1. GENERAL EXPERIMENTS

### a. ESTABLISHMENT OF IPS CELL LINES AND DIFFERENTIATION OF CARDIAC CELL TYPE

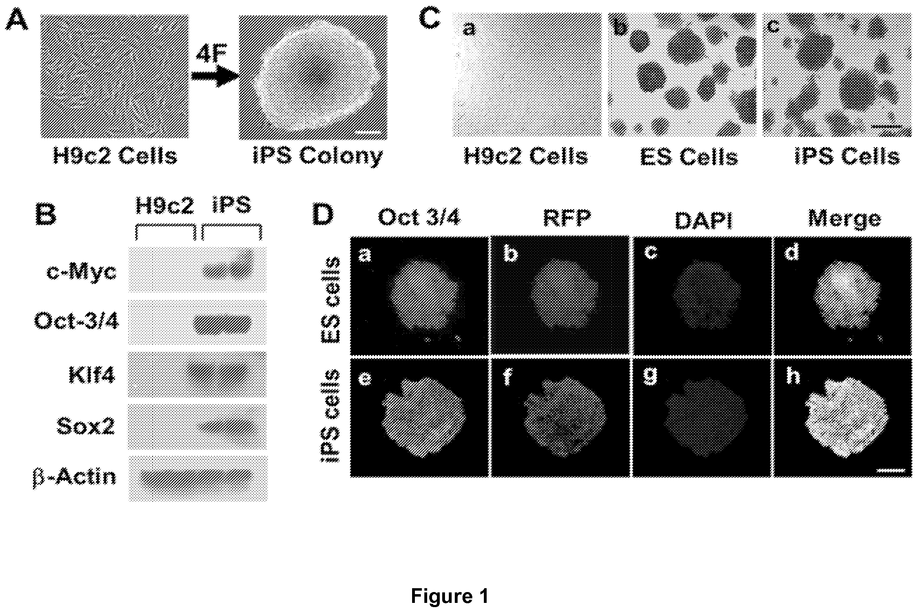

Stably transfected iPS cell lines expressing red fluorescence protein (RFP) were generated. (Singla et al., 2011). H9c2 cells were transfected with an expression vector expressing mouse sternness four factors (i.e., the 4F expression vector). The four sternness factors were c-Myc, Oct 3/4, Klf4 and Sox2. Figure 1A shows photomicrographs of untransduced H9c2 cells and shows transduced H9c2 cells forming ES cell like colony (right panel) (scale bar = 50 µm). Untransduced H9c2 cells appear elongated, but following transduction with the 4F expression vector, these H9c2 cells appeared morphologically distinct from the untransduced H9c2 cells (Figure 1A, left panel vs. right panel).

To determine whether these four factors were present in the generated iPS cells, western blot analysis was utilized to show expression for these four sternness factors in the iPS cells. As shown in Figure 1B, western blot analysis did not show the presence of any one of the four factors (c-Myc, Oct3/4, Klf4 and Sox2) in the untransfected H9c2 cells, but increased expression of four factors was detected in the transduced H9c2 cells.

Next, whether generated iPS cells expressed pluripotency markers, such as alkaline phosphatase and Oct3/4, was next determined. As shown in Figure 1C, iPS cells expressed alkaline phosphatase in 100% of growing cells (left panel). Similar expression was observed in ES cells, which were used as a positive control (middle panel). In contrast, untransduced H9c2 cells were negative for alkaline phosphatase staining (right panel) (scale bar = 250 µm). Moreover, as seen in Figure 1D, growing iPS cells were positive for the undifferentiated pluripotent marker Oct3/4 (which was compared with ES cells as a positive control). Figure 1D also shows RFP expressing ES and iPS cells (b and f); DAPI staining for ES and iPS cells (c and g), and a merged image Oct3/4, RFP, and DAPI of ES and iPS cells (d and h). These data indicate that generated iPS cells were reprogrammed and expressed pluripotent markers, (scale bar = 100 µm). (Singla et al., 2011).

Whether the iPS cell lines retained the capability in vitro to differentiate into all three major heart cell types (i.e., cardiomyocytes, vascular smooth muscle cells and endothelial cells) present and still express the desired reporter protein was evaluated.

### b. FGF-8 ENHANCEMENT OF CARDIAC MYOCYTE DIFFERENTIATION FROM IPS CELLS

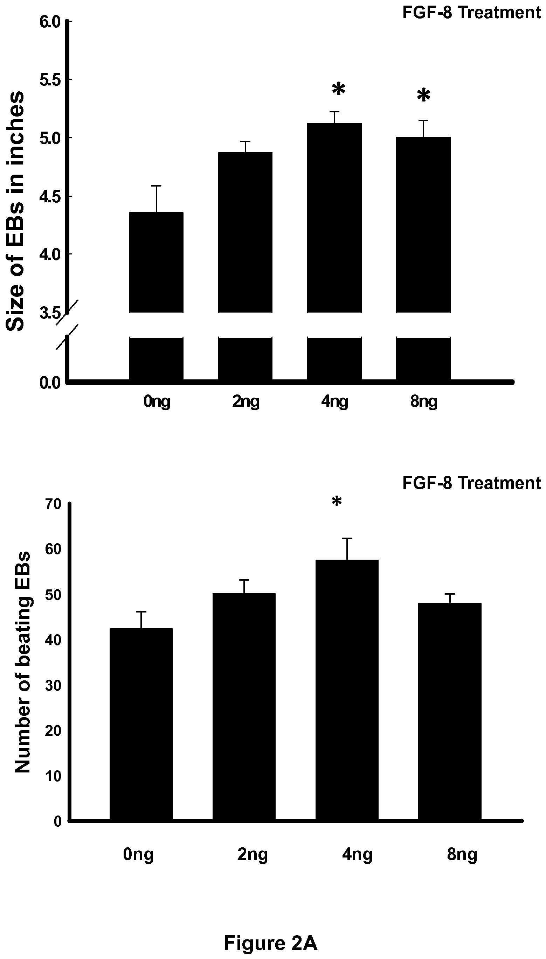

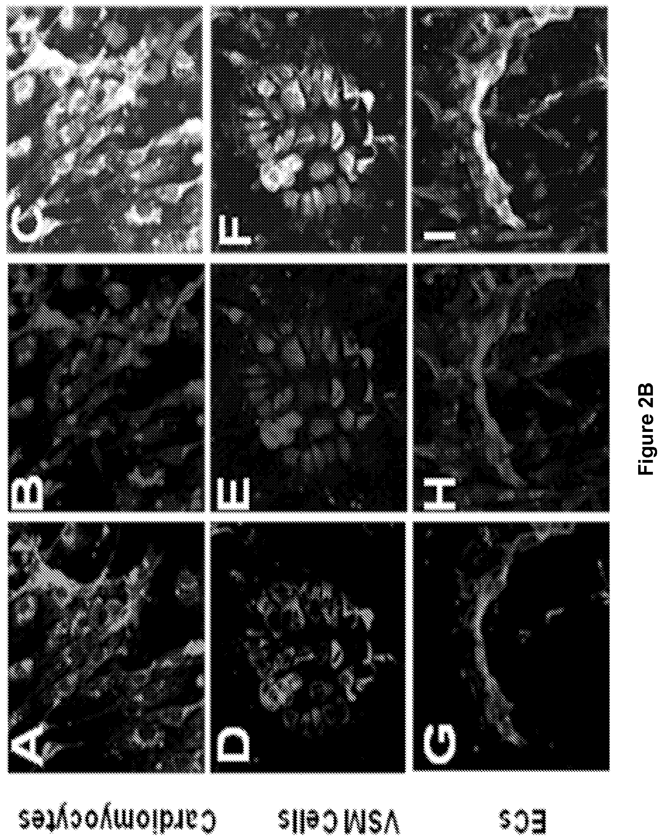

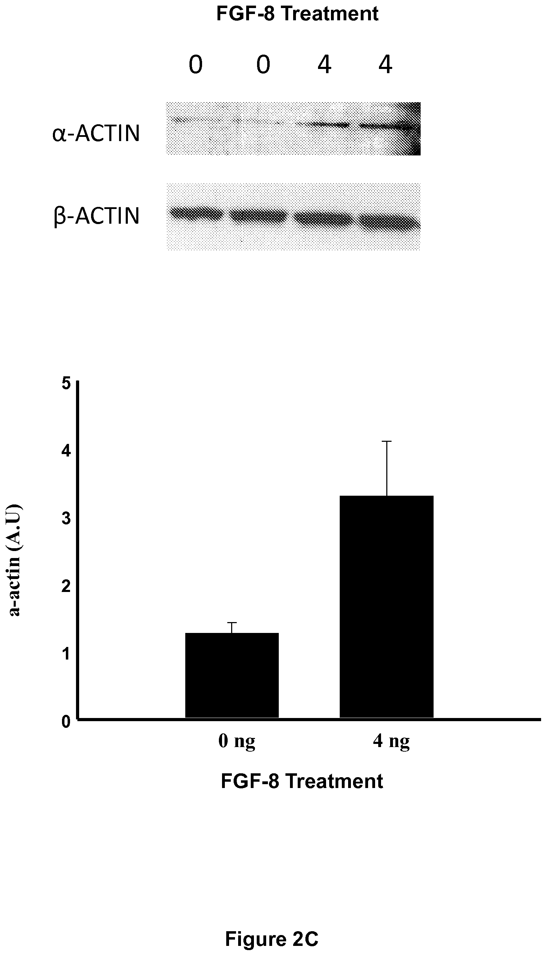

The ability of iPS cell-derived EB cell culture system to produce beating cardiac myocytes was confirmed. Whether direct treatment of iPS cells with FGF-8 promoted differentiation into beating cardiac myocytes. EBs derived from iPS cells were treated with 0, 2, 4, and 8 ng per mL of the bio-active form of recombinant human FGF-8 and examined by microscopy from day 0 (D0) through day 17 (D17) after plating. Figure 2A (top panel) shows the size of EBs in inches following treatment with FGF-8. Figure 2A (bottom panel) shows the number of beating EBs following the same course of FGF-8 treatment. Figure 2B shows that iPS cells form embryoid bodies (EBs) and differentiate into cardiac myocytes, smooth muscle cells and endothelial cells. EBs were differentiated for 17 days. A beating area (i.e., cardiac myocytes) was stained with anti-myosin/Alexa 488 (2B-A), anti-RFP/Alexa 568 (2B-B), and DAPI (2B-C). The merged image (2B-C) shows co-expression of myosin and RFP. Smooth muscle cells were stained with anti-smooth muscle actin/Alexa 488 (2B-D), anti-RFP/Alexa 568 (2B-E), and DAPI (2B-F). The merged image (2B-F) shows overlap of expression of smooth muscle α-actin and RFP. Endothelial cells that have formed a capillary-like structure were stained with anti-von Willebrand Factor/Alexa 488 (2B-G), anti-RFP/Alexa 568 (2B-H), and DAPI (2B-I). The merged image (2B-I) shows co-expression of von Willebrand Factor and eGFP. Figure 2C (top panel) shows the image of a western blot using anti-α-actin and anti-β-actin following FGF-8 treatment (0 ng per mL and 4 ng per mL). Figure 2C (bottom panel) shows the quantification of the western blot data.

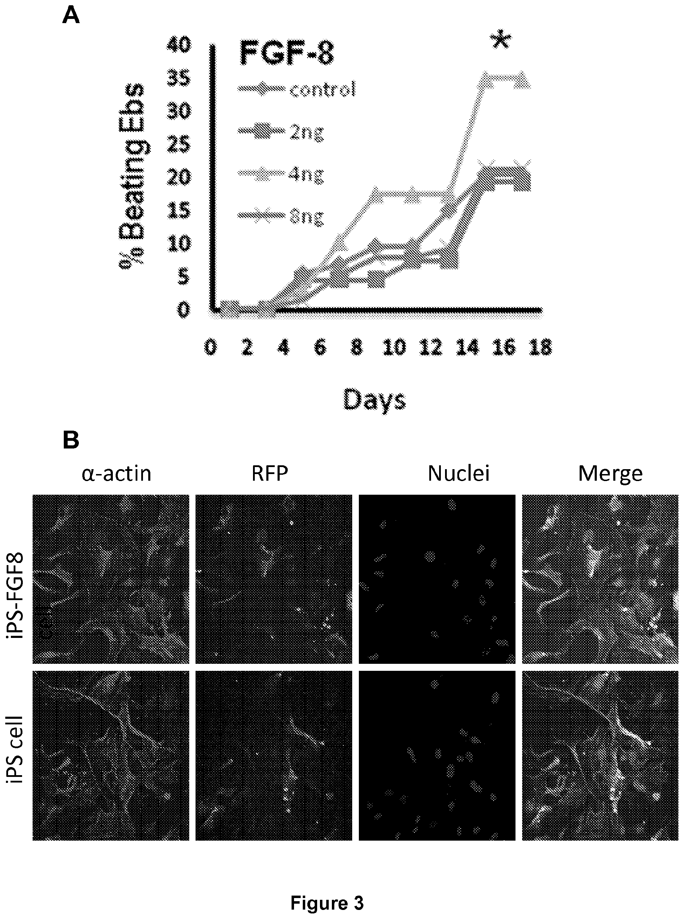

FGF-8 significantly increased EB proliferation by 20-30% on D5 as compared to the controls (p < 0.05). In Figure 3A, the results are plotted as a percentage increase in beating EBs at D0-D17. The percentage of rhythmically beating EBs after treatment with FGF-8 (4 ng) was significantly greater compared with controls (37% versus 20%, at D17 after plating, *p < 0.05). The data are from the sets of three-five independent experiments. Figure 3B shows the staining of iPS cells treated with FGF-8 (top panel) and iPS cells not treated without FGF-8 with anti-α-actin and anti-RFP antibodies. 29.2% of cells that stained positively for α-actin from beating area.

### c. ESTABLISHMENT OF APOPTOTIC CELL CULTURE MODEL AND DETERMINATION OF EFFECTS OF IPS-CM AND FGF-8 ON APOPTOSIS

The effects of iPS-CM on induced apoptosis cells by H₂O₂ were examined. The concentrations of H₂O₂ required for induction of apoptosis in H9c2 cells was determined. H9c2 cells treated with 50-400 µm of H₂O₂ demonstrated significant decrease in cell survival at the tested concentrations. The observed maximum decrease in cell survival (58%) occurred at 400 µm H₂O₂ (p < 0.05) (Singla et al., 2007; Singla et al., 2008).

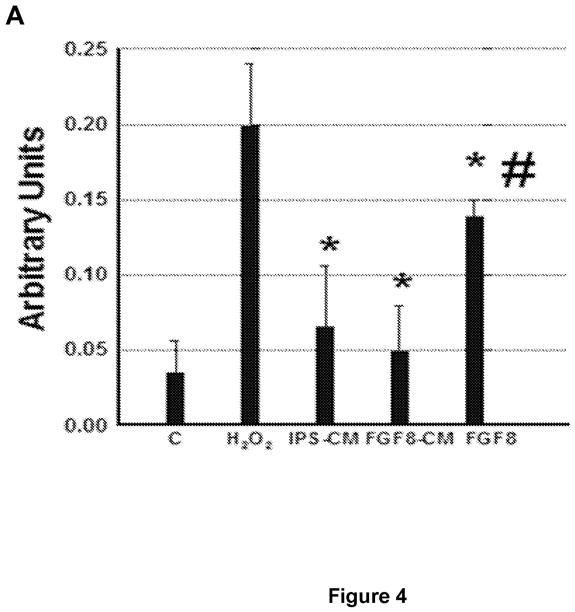

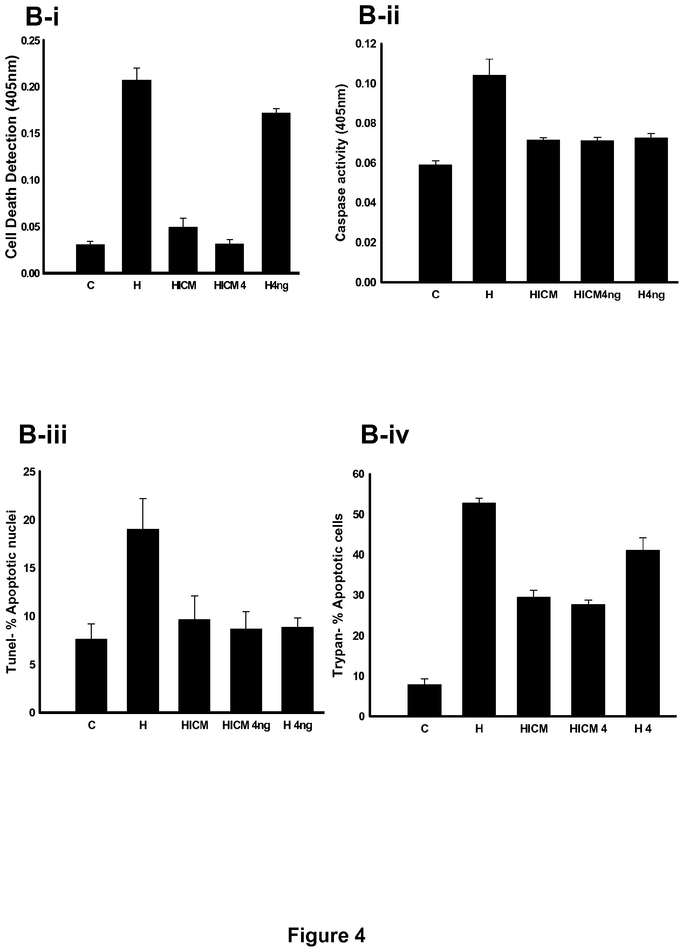

Using quantitative apoptotic ELISA method (Singla et al., 2007; Singla et al., 2008), the effect of iPS-CM prepared using both (i) with or (ii) without FGF-8 treated iPS cells on H₂O₂ induced apoptosis in H9c2 cells. The histograms of Figure 4A shows a significant increase in DNA fragmentation as measured by apoptotic ELISA (Singla et al., 2007) in H₂O₂ treatment when compared with untreated control group (p <0.05). The amount of cell death induced by H₂O₂ was significantly reduced (p < 0.05) following treatment with both iPS-CMs (Figure 4). Next, whether FGF-8 independently inhibited H₂O₂ induced apoptosis in this model was determined. Cell were treated with 4 ng of FGF-8 following H₂O₂ treatment. FGF-8 alone significantly reduced apoptosis. Treatment with both iPS-CMs generated a more robust reduction in apoptosis then treatment with FGF-8 alone (Figure 4). Data are from the set of 8-10 independent experiments. This data indicated that FGF-8 as well as iPS-CMs prepared from iPS cells with or without FGF-8 treatment act as an anti-apoptotic factor as well.

The role of FGF-8 in H₂O₂ induced apoptosis in H9c2 cells was evaluated. FGF-8 demonstrated significant (p < 0.05) inhibition of H₂O₂ induced apoptosis in H9c2 cells. This was confirmed by apoptotic ELISA (Figure 4A). The inhibition of apoptosis by iPS-CM as determined with apoptotic ELISA kit was significantly lower (p < 0.05) than the amount inhibited by FGF-8 alone (Figure 4). The apoptotic ELISA data indicate the presence of factors other than FGF-8 that are also released into iPS-CMs and contribute in the inhibition of H₂O₂ induced apoptosis in H9c2 cells.

Figure 4B shows cell death detection (4B-i), caspase activity (4B-ii), tunnel % apoptotic nuclei (4B-iii), and trypan % apoptotic cells (4B-iv) following treatment with H₂O₂ (H); or H₂O₂ + conditioned medium (HICM); or H₂O₂ + conditioned medium + 4 ng of FGF-8 (HICM4ng); or H₂O₂ + 4 ng of FGF-8 (H4ng). Control is indicated as C.

Those cytokines and/or growth factors present in the iPS-CMs that can provide protection from H₂O₂-induced apoptosis were identified. 69 cytokine and/or growth factors were analyzed with the use of Luminex technology (Singla et al., 2007). Cytoprotective proteins released into both iPS-CM and FGF-8-iPS-CM were present at higher levels as compared with H9c2-CM, ES-CM, or cell culture media used as a control. Three major proteins known to inhibit apoptosis and enhance cardiac cell proliferation in the embryonic hearts were identified (Table 5).

**Table 5. Identification of Factors Released From iPS-CM**

| **Released Factors** | **Control #** | **H9c2 Cells-CM** | **ES Cells-CM** | **iPS-CM** | **FGF-8-iPS-CM** | **Comments** |
|---|---|---|---|---|---|---|
| **FGF-9** | L* | 140^{$} | 140 | 370 | 370 | Pro-cardiac growth factor in embryonic heart |
| **IL-10** | L | L | L | 45 | 84 | Anti -apoptotic factor in cardiac myocytes |
| **TIMP-1** | 7 | 19 | 130 | 380 | 380 | Anti-apoptotic; also well known as anti-fibrotic |
| **FGF-8** | Pro-cardiac growth factor in the embryonic heart; its anti-apoptotic and pro-cardiac characteristics in apoptotic cells and in iPS-EB cell culture system established herein | | | | | |
| ^{$}Values are expressed as pg/mL | | | | | | |
| *L=lowest detectable dose by the assay | | | | | | |
| ^{#}Cell culture medium used to grow ES cells | | | | | | |

### d. ESTABLISHMENT OF THE MYOCARDIAL INFARCTION MODEL AND CARDIAC REGENERATION

Myocardial infarctions (MIs) were produced in C57BL/6 mice and iPS, ES, H9c2 cells, or cell culture medium were transplanted post-MI. (Singla et al., 2011; Fatma et al., 2010; Kumar et al., 2005) Immediately after coronary ligation, two intramyocardial injections of 10 µL of medium ± 2.5 x 104 iPS cells were delivered into infarct border zones.(Singla et al., 2011; Fatma et al., 2010). LV of MI + cell culture medium (n = 8 in each group) group were compared with MI + iPS cells, MI + ES cells, and MI + H9c2 cells. MI groups were transplanted with cell culture medium as a control. Hearts were removed and examined at 2 weeks after cell transplantation.

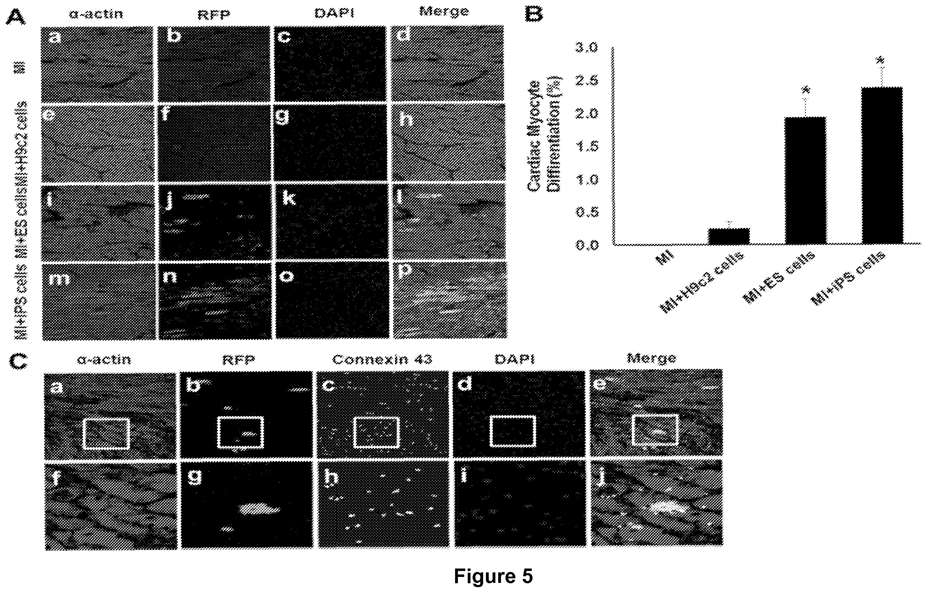

Cardiac myocyte specific anti-sarcomeric α-actin antibody was used to identify cardiac myocytes (Figure 5A; panels a, e, I, and m), anti-RFP antibody was used to identify donor cells (Figure 5A; panel b, f, j, and n), and DAPI staining identified nuclei (Figure 5A; panels c, g, k, and o). Merged images of all three stainings are shown in Figure 5A (panels d, h, l, and p). Heart sections stained with an anti-RFP antibody demonstrated that the transplanted iPS cells were present in the peri-infarct and infarct regions of the myocardium in C57BL/6 mice (Figure 5A; panel n). There were no stained cells in H9c2 cell transplanted hearts (Figure 5A; panel f), MI + ES or iPS cell transplanted hearts stained with an anti-RFP antibody demonstrated patchy areas of engrafted cells in the peri-infarct and infarct region of the heart (Figure 5A; panels j and n). In Figure 5A, the scale bar = 50 µm.

To confirm whether transplanted cells differentiated into cardiac myocytes, all four groups were co-labeled with a sarcomeric α-actin antibody. Colocalization of cardiac actin and RFP was demonstrated in both cell transplanted sections, which indicated that the transplanted stem cells differentiate into new cardiac myocytes (Figure 5A; panels d, h, l, and p). Newly generated cardiac myocytes were rarely found in the H9c2 cell transplanted group. Quantitative data shows that there were 1.92 ± 0.3% newly formed cardiac myocytes in ES cell transplanted hearts as compared to 2.37 ± 0.3% in iPS cells (Figure 5B). The percent positive differentiated cardiac myocytes in iPS and ES groups were significantly (p < 0.05) different compared to the H9c2 cells and MI groups (*p < 0.001 vs. MI and H9c2 cells). There was no statistical difference between iPS and ES cell groups (Figure 5B).

To confirm whether newly generated cardiac myocytes demonstrate electrical coupling in the native myocardium, heart sections were triple immunolabeled (i.e., RFP to detect donor iPS cells (panels b and g), sarcomeric α-actin to detect cardiac myocytes (panels a and f), and connexin-43 to identify gap junctions (panels c and h). DAPI staining to identify nuclei is shown in Figure 5C (panels d and i) and the merged image is shown in panels e and j. Figure 5C shows newly generated cardiac myocytes form gap junctions, were electrically coupled, and integrated into the myocardium. (Singla et al., 2011). In Figure 5C (panels a-e), the scale bar scale bar = 50 µm, and for panels (f-j), the scale bar = 20 µm.

### e. INHIBITION OF APOPTOSIS BY THE TRANSPLANTATION OF IPS CELLS

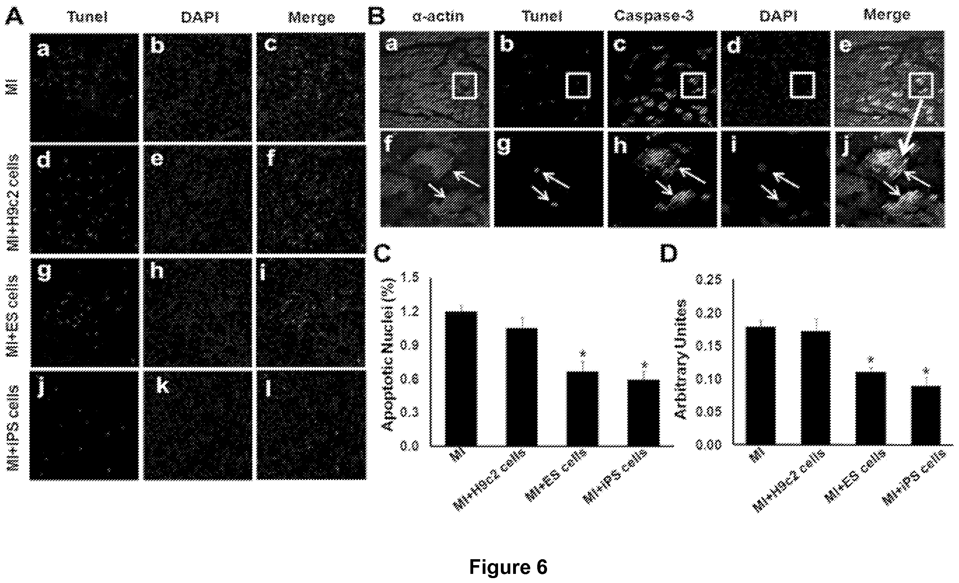

Effects of transplanted iPS cells on cardiac apoptosis were determined by TUNEL staining, caspase-3 immunostaining, and caspase-3 activity. (Singla et al., 2011). TUNEL staining data showed a reduced amount of red apoptotic nuclei in MI + iPS or ES cell groups as compared to the wide spread red apoptotic nuclei in MI and MI + H9c2 cell groups (Figure 6A). DAPI stained total nuclei (Figure 6A; panels b, e, h, and k) and merged images are visible (Figure 6A; panels c, f, i and 1). In Figure 6A, the scale bar = 50 µm.

Figure 6B shows anti-sarcomeric α-actin staining (Figure 6B; panels a and f), TUNEL (Figure 6A; panels b and g), caspase-3 immunolabeling, (Figure 6B; panels c and h), and DAPI (Figure 6B; panels d and i). Merged images are shown in Figure 6B (panels e and j). In panels a-e, the scale bar = 50 µm and in panels f-j, the scale bar = 20 µm.

Quantitative TUNEL apoptotic nuclei showed transplanted iPS cells significantly (p < 0.05) reduced apoptotic nuclei (0.6 ± .07%) as compared to the MI or MI + H9c2 cell groups (1.2 ± 0.1% apoptotic nuclei/section for MI; 1 ± 0.2% apoptotic nuclei/section for MI ± H9c2 cells; Figure 6C). Transplanted ES cells reduced apoptosis comparable to the iPS cell group. TUNEL stained heart sections were also combined with caspase-3 staining to confirm cell death was apoptotic in nature.

Some of the sections were also co-stained with a third antibody, sarcomeric α-actin, to confirm apoptosis occurred in the cardiomyocytes. The data in Figure 6B confirms apoptotic nuclei were stained with TUNEL, were co-labeled with caspase-3 antibody, and were positive with sarcomeric α-actin, which indicated that apoptosis occurred in cardiac myocytes. Next, caspase-3 activity, which is considered a hallmark of apoptosis, was measured. The data showed that following iPS or ES cell transplantation, caspase-3 activity was significantly (p < 0.05) reduced as compared to MI, and that H9c2 cells demonstrated no decrease in caspase-3 activity (Figure 6D). The caspase-3 activity data coupled with the TUNEL apoptotic nuclei staining confirmed apoptosis in the heart.

### f. INHIBITION OF FIBROSIS BY TRANSPLANTED IPS CELLS

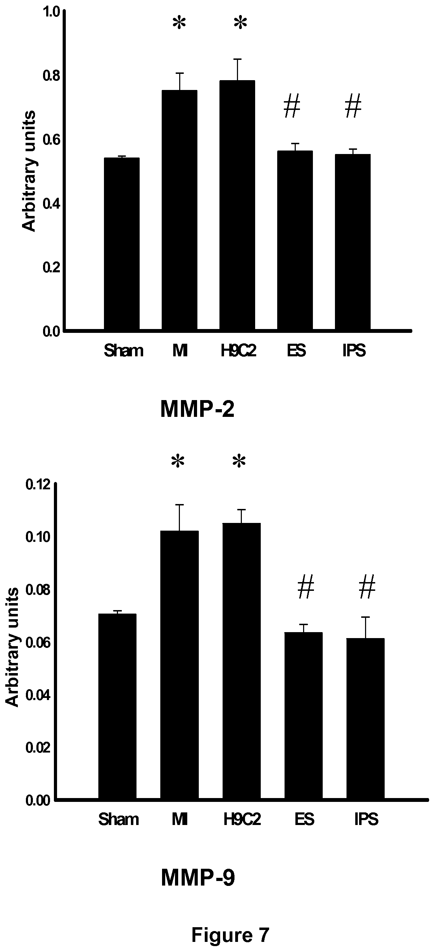

To determine whether inhibition of fibrosis was associated with changes in MMP-2 and MMP-9, both of which are well-documented MMPs involved in the pathogenesis of MI, the levels of MMP-2 and MMP-9 were quantified using an enzyme-linked immunoassay. As shown in Figure 7 (top panel), hearts transplanted with ES and iPS cells demonstrated significantly reduced MMP-2 concentration as compared to MI and H9c2 cell controls (*p < 0.05 vs. sham, #p < 0.05 vs. MI and MI + H9c2). Figure 7 (bottom panel) shows that hearts transplanted with ES and iPS cells demonstrated significantly reduced MMP-9 concentration as compared to MI and H9c2 cell controls. (*p < 0.05 vs. sham, #p < 0.05 vs. MI and MI + H9c2).

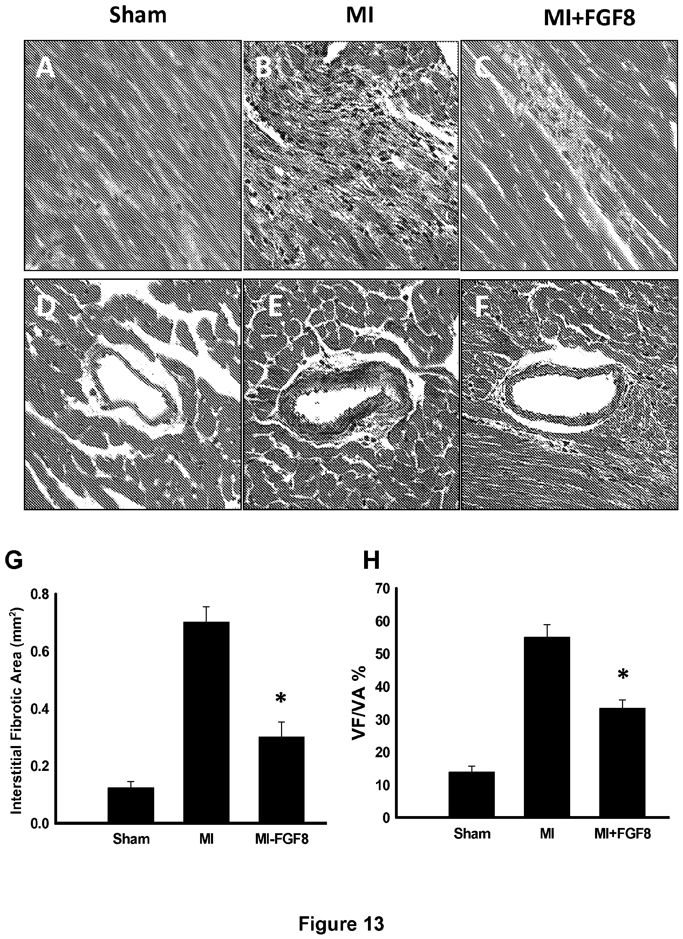

Also, Figures 13A-E show histological staining (trichome staining) demonstrating the effect of sham-MI, MI, and MI with FGF-8 treatment. Figure 13G shows the volume of interstitial fibrotic area. Figure 13H shows the comparison of vascular fibrosis (VF) vs. vascular area (VA) (in %) following sham-MI, MI, and MI with FGF-8 treatment.

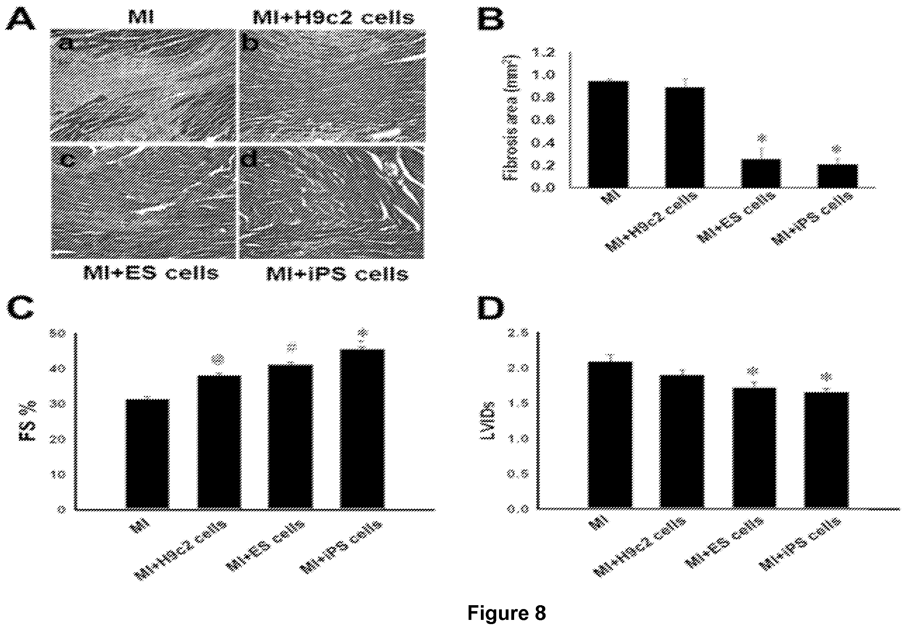

Histological examinations were then performed to determine whether transplanted iPS or ES cells inhibited adverse cardiac remodeling (Singla et al., 2011). Figure 8A shows representative photomicrographs from Masson's trichrome stained heart sections from the various treatment groups. LV remodeling was determined by measuring total interstitial fibrotic area in mm² in all mice groups transplanted with or without stem cells. Heart sections obtained from infarcted hearts transplanted with iPS and ES cells showed a significant (p < 0.05) decrease in fibrosis (0.25 ± 0.09 mm² for MI + iPS cells and 0.21 ± .05 mm² for MI+ES cells) as compared to the large fibrotic areas in MI and H9c2 cell transplanted hearts (0.94 ± 0.03 mm² for MI and 0.9 ± 07 mm² for MI + H9c2 cells, Figure 8B).

### g. ECHOCARDIOGRAPHIC EVIDENCE OF IMPROVEMENT IN LV FUNCTION WITH CELL TRANSPLANTATION

M-mode echocardiography was used to determine the effect of cell transplantation on LV size and function in mice two weeks post-MI. Fractional shortening (FS) was improved (*p < 0.05) by H9c2 cell transplantation, ES cells, and cardiac iPS cells. (Singla et al., 2011). iPS cells showed a robust increase in FS as compared to H9c2 cells (*p < 0.05 vs. H9c2 and ES cells) and as compared to MI + cell culture medium (#p < 0.001 vs. MI) (Figure 8C and 8D).

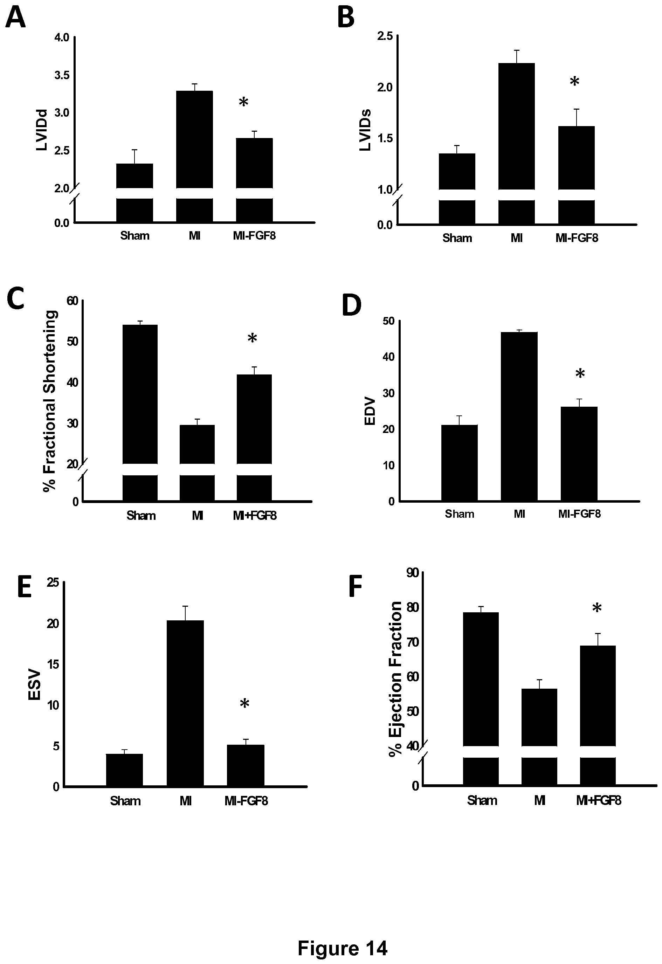

Moreover, Figure 14A-F shows the results of various outcome measures including fractional shortening and % ejection fraction following sham-MI, MI, and MI with FGF-8 treatment. (LVIDd = left ventricular internal diameter in diastole; LVIDs = left ventricular internal diameter in systole; EDV = end-diastolic volume; ESV = end-systolic volume). These data are quantified in Table 6

**Table 6. Cardiac Function**

| Group | LVIDd | LVIDs | EDV | ESV | FS | EF |
|---|---|---|---|---|---|---|
| Sham | 2.319 ± 0.19 | 1.346 ± 0.08 | 21.148 ±2.60 | 3.940 ± 0.62 | 53.927 ± 1.11 | 78.364 ± 1.85 |
| MI | 3.283 ± 0.1 | 2.330 ± 0.13 | 46.776 ± 0.69 | 20.288 ± 1.77 | 29.419 ± 1.54 | 54.386 ± 2.11 |
| MI-FGF-8 | 2.654 ± 0.1 | 1.61 ± 0.17 | 26.132 ± 2.26 | 5.082 ± 0.73 | 43.619 ± 2.51 | 68.712 ± 3.78 |

### h. INHIBITION OF APOPTOSIS VIA TRANSPLANTED FGF-8

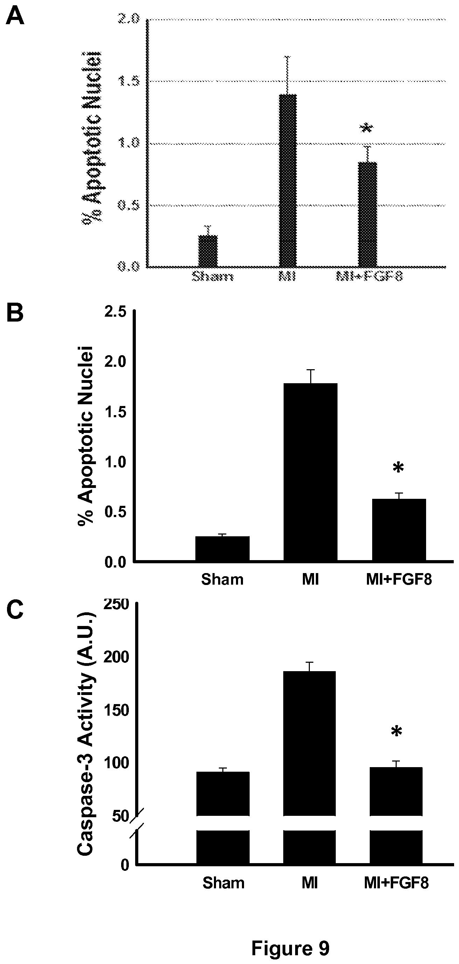

The cell culture data indicated that FGF-8 is a novel anti-apoptotic factor (see Figure 4). Two intramyocardial injections of 20 µL FGF-8 containing 50 ng/mL were performed. Hearts were removed and examined at 2 weeks post-MI. As shown in Figure 9A and Figure 9B, TUNEL staining showed that apoptosis post-MI was significantly reduced in the FGF-8 treated MI hearts as compared to non-FGF-8 treated MI hearts (*p < 0.05). Figure 9C shows the casapse-3 activity under the same experimental conditions.

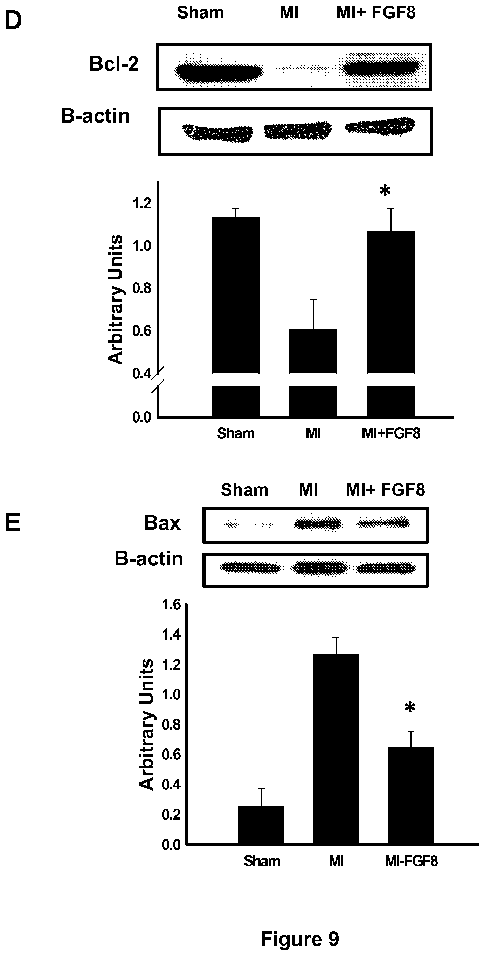

Furthermore, Figure 9D and Figure 9E shows the effect of FGF-8 treatment on the expression of Bcl-2 and Bax. Briefly, the Bcl-2 family consists of more than twenty anti- and pro-apoptotic members that modulate the balance between life and death. Figure 9D (top panel) shows western blotting of sham, MI, and MI+FGF-8 treated samples with anti-Bcl-2 and anti-beta-actin (control) antibodies. Figure 9D (bottom panel) shows the quantitative results of the western blotting experiments involving Bcl-2 (n = 5). Figure 9E (top panel) shows western blotting of sham, MI, and MI+FGF-8 treated samples with anti-Bax and anti-beta-actin (control) antibodies. Figure 9E (bottom panel) shows the quantitative results of the western blotting experiments involving Bax (n = 3).

### i. EFFECT OF TRANSPLANTED STEM CELLS ON MIR-486 EXPRESSION

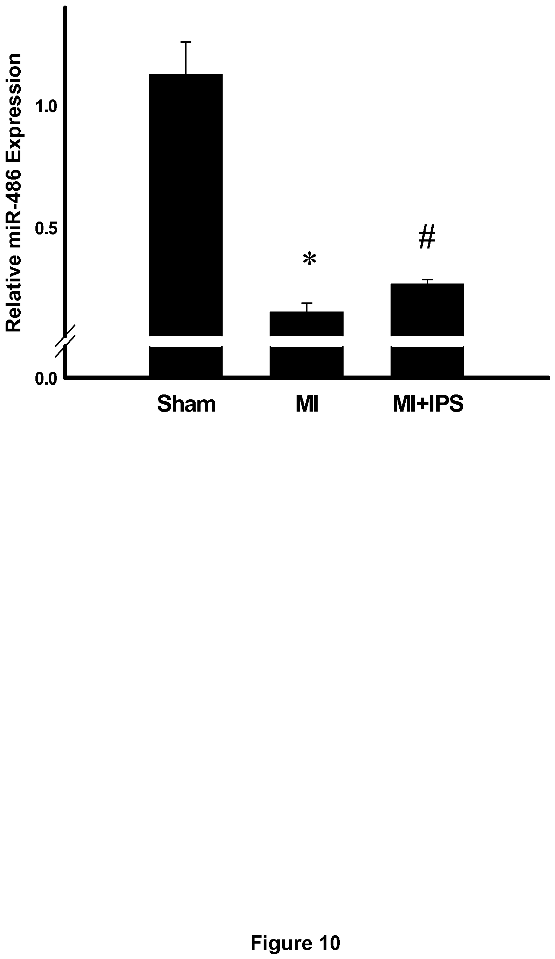

The MI heart data indicated a significant increase in cardiac apoptosis. This increase was blunted following iPS cell transplantation (see Figure 6). A miRNA signature panel of 365 different miRNAs (rodent taqman array) was used to determine up- or down-regulation of various miRNAs with and without cell transplantation post-MI. As shown in Figure 10, the levels of miR-486 were significantly decreased following MI and this decrease was inhibited following iPS cell transplantation), indicating that miR-486 is a cell survival miRNA.(*p < 0.001 vs. sham, #p < 0.05 vs. MI).

### j. EFFECT OF TRANSPLANTED IPS CELLS ON AKT AND PTEN PATHWAYS

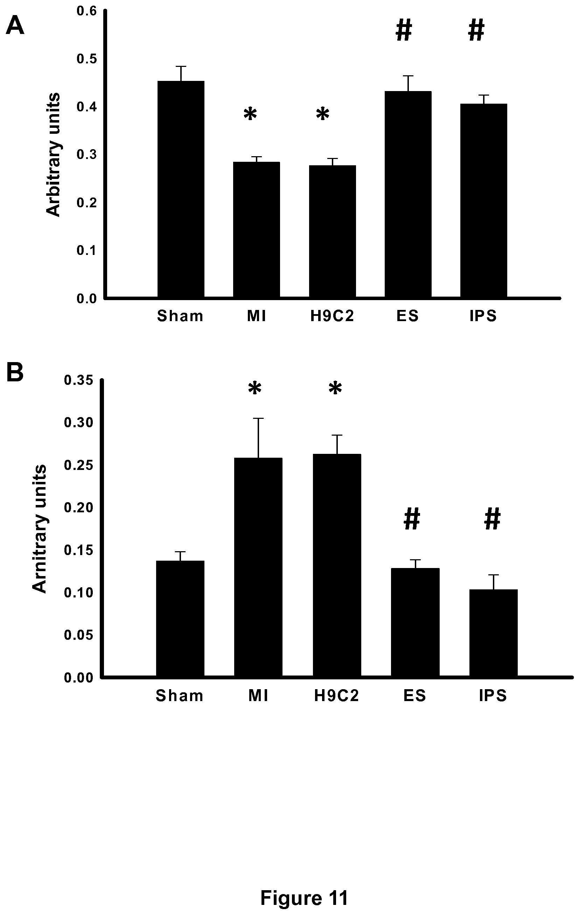

Direct and indirect mechanisms of apoptotic inhibition can occurs through activation of various cell survival cascades such as adenosine triphosphate-dependent tyrosine kinase (Akt). Using a phosho-Akt ELISA, it was determined that hearts transplanted with ES and iPS cells had significant activation of p-Akt as compared to hearts transplanted with H9c2 cells and cell culture medium (Figure 11A, *p < 0.05 vs. sham, #p < 0.05 vs. MI and MI + H9c2). To evaluate whether increased activation of p-Akt was attributable to inhibition of phosphoinositide-3-kinase (PI3K)/Akt signaling, levels of phosphatase and tensin homolog (PTEN), an inhibitor of PI3K phosphorylation, were quantified. This data showed a significant reduction in PTEN concentration in hearts transplanted with ES and iPS cells as compared to hearts transplanted with H9c2 cells or cell culture medium (Figure 11B, *p < 0.05 vs. sham, #p < 0.05 vs. MI and MI + H9c2).

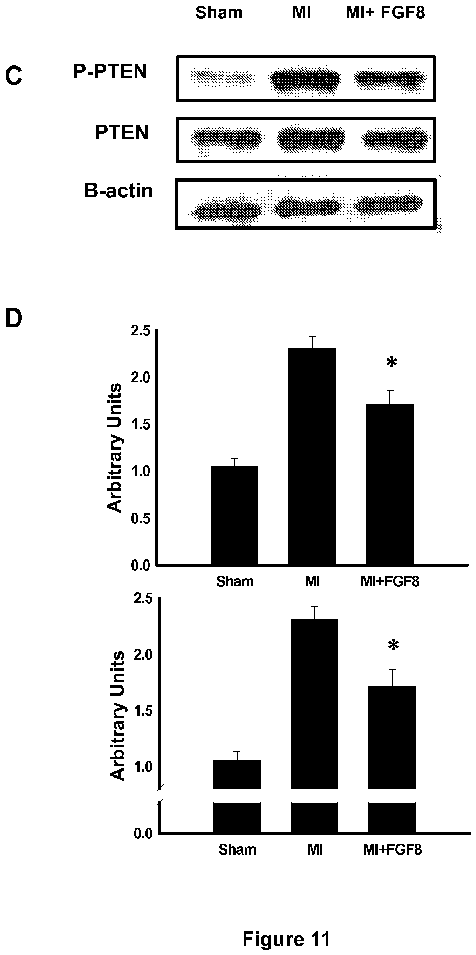

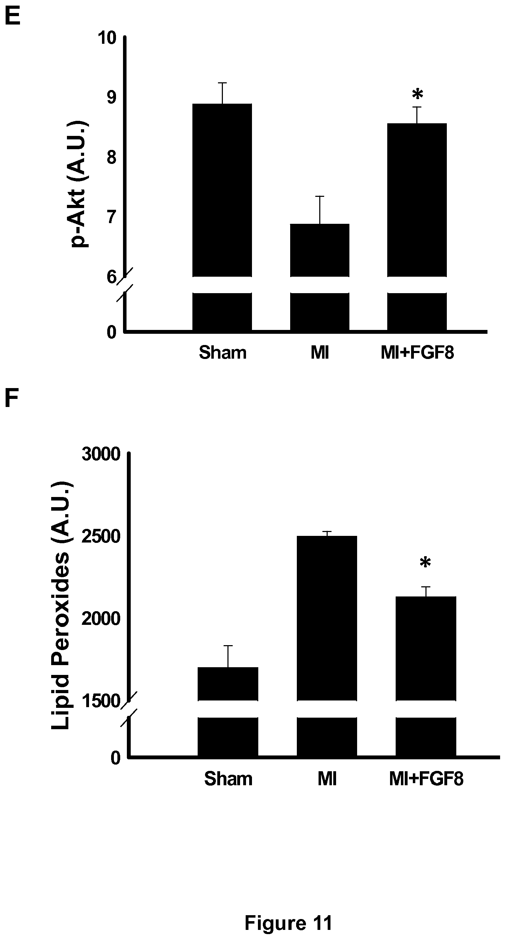

The effect of FGF-8 treatment on PTEN and P-PTEN expression in heart homogenates two weeks after either sham-treatment (sham) or myocardial infarction (MI) or myocardial infarction with FGF-8 treatment (MI+FGF8) is shown in Figures 11C and 11D. Figure 11C shows a western blot of sham, MI, and MI+FGF-8 utilizing anti-PTEN, anti-P-PTEN, and anti-Beta-actin antibodies. Figure 11D (top and bottom panels) shows the quantitation of these western blot experiments. Figure 11E shows p-AKT expression in following sham treatment (sham), myocardial infarction (MI), and myocardial infarction with FGF-8 (MI+FGF8). Figure 11F shows lipid peroxidase expression following sham treatment (sham), myocardial infarction (MI), and myocardial infarction with FGF-8 treatment (MI+FGF8).

### 2. DETERMINATION OF WHETHER TRANSPLANTATION OF IPS CELLS PRIMED WITH FGF-8 ENHANCES CARDIAC REPAIR AND REGENERATION FOLLOWING MI

### a. GENERAL METHODS

Cell therapy is currently being examined for angiogenesis, cardiac regeneration, and repair in MI. (Singla et al., 2011; Fatma et al., 2010; Behfar et al., 2002; Balsam et al., 2004; Beltrami et al., 2003; Shujia et al., 2008). Success in cell transplantation requires an understanding of the molecular mechanisms of improved cardiac function and identification of appropriate growth factor or factors to enhance engraftment and proliferation following cell transplantation. Certain growth factors such as granulocyte-colony stimulating factor and stem cell factor have been shown to enhance myocardial regeneration. (Orlic et al., 2001). Mouse ES cells in the presence of TGFβ2 have been shown to enhance cardiac myocyte differentiation. (Singla et al., 2005).

FGF-8 is a predominant cardiac growth factor, which plays a role in the development of heart. (Ilagan et al., 2006; Marques et al., 2008; Reifers et al., 2000). FGF-8 is a growth factor which plays a role in heart development as explained above. (Ilagan et al., 2006). To date, the role of FGF-8 in adult heart regeneration or with iPS cell treatment has never been explored. However, as demonstrated herein, FGF-8 (i) acts as an anti-apoptotic factor in the adult infarcted heart, and (ii) activates iPS cells and stimulates differentiation into cardiac myocytes.

### (1) DETERMINATION OF IPS CELL PROLIFERATION AND CELL DIVISION POST-MI

The determination of the number of engrafted iPS cells which have started proliferating is as follows: BrdU (50 mg/kg body weight, i.p. (Beltrami et al., 2003), which identifies nuclei in S phase in order to label actively dividing cells, is administered to the subject. Active cell growth of engrafted cells is identified using double-label immunostaining for RFP and BrdU (Dako). RFP identifies donor engrafted cells and BrdU identifies DNA synthesizing cells. To determine how many cells have started cell division at any given time, Ki-67 is utilized. Ki-67 is a nuclear antigen that is associated with cell division and that labels proliferating cells (G1-, S-, G2-phase and mitosis), but does not label quiescent or resting cells (GO-phase). Double-label immunostaining is used to detect proliferating cells using Ki-67 (Dako) antibodies and donor engrafted cells using RFP antibodies. Slides are analyzed using fluorescence (Olympus) and confocal microscopy.

### (2) IDENTIFICATION OF CELL TYPE DIFFERENTIATION POST-MI

Cell differentiation is determined using double-label immunostaining to detect donor cells (RFP antibody) and differentiated cell types using cell specific antibodies. Cardiac-specific antibodies such as cardiac myosin heavy chain (MF-20 antibody, hybridoma bank), sarcomeric α-actin (Sigma), α-actinin, cardiac troponin I (Spectral Diagnostic), and connexin-43 (Chemicon) are used to detect cardiac myocytes and gap junctions. Endothelial cells are detected with factor VIII (Sigma) and Griffonia simplicifolia lectin (Sigma) antibodies. For smooth muscle cells, α-smooth muscle actin antibody (sigma) is used. To determine the newly formed progenitor cardiac myocytes, sections are immunostained with cardiac specific transcriptional factors such as GATA-4, Nkx2.5, and MEF2 (Santa Cruz Biotechnology). Sections are then stained with respective FITC or rhodamine conjugated secondary antibodies and counterstained with DAPI for nuclear visualization. Slides are analyzed using Olympus fluorescence and confocal microscopy.

### (3) IDENTIFICATION OF MMPS ASSOCIATED WITH FIBROSIS

SDS-PAGE and Western blot analyses are utilized to determine the protein expression of collagen types I and III (Santa Cruz Biotechnology), MMP-2 and MMP-9 (Santa Cruz Biotechnology/Cell Signaling), and TIMP-1 (Santa Cruz Biotechnology). Densitometry is used to measure band density. ELISA is performed with a kit (R & D, Minneapolis) to measure MMP-2 and MMP-9 in the LV tissue homogenates.

### (4) FUNCTIONAL ASSESSMENT OF THE REGENERATED HEART

Echocardiography is an accurate noninvasive tool for the determination of quantitative characterization of post- MI heart remodeling in the mouse model. (Singla et al., 2011; Fatma et al., 2010). Echocardiography is done on all groups post-MI hearts at short-term and long-term time points. M-mode images (Singla et al., 2011; Fatma et al., 2010) in a short axis view is performed to measure LV mass, LV mass to body ratio, LV anterior and posterior wall thickness, and fractional shortening.

### (5) AVOIDANCE OF TERATOMA FORMATION

Both human and mouse ES cells form complex teratomas when engrafted into an immune deficient host, a characteristic of ES cells resulting from their pluripotential capacity (Damjanov et al., 2004; Thomson et al., 1998). Teratoma formation is considered a major potential limitation in the therapeutic use of ES cells, especially when the transplanted undifferentiated number of cells is higher than 100,000 (Nussbaum et al., 2007). It has been suggested that intramyocardial transplantation of 1x10⁶ ES or iPS (generated from fibroblasts) cells in the mouse heart causes teratomas (Nelson et al., 2009; Nussbaum et al., 2007). However, no teratoma formation was observed with transplantation of 300,000 cells (Behfar et al. 2002). The transplantation of 3x10⁴ undifferentiated ES cells did not result in the formation of teratomas after 2 (Singla et al., 2006) and 4 weeks. No teratoma formation in the infarcted heart following H9c2 cell-induced iPS cell transplantation (5x10⁴ undifferentiated iPS cells) was observed. (Singla et al., 2011). This number of transplanted iPS cells was less than that in studies reporting teratoma formation (Nussbaum et al., 2007) (range varies from 100,000 to one million undifferentiated ES or iPS cells). In an effort to avoid teratoma formation, the experiments discussed below utilize 1 x 10⁵ differentiated iPS cardiac committed (FGF-8 primed) cells. If a larger number of transplanted iPS cells are required by methods disclosed herein, then teratoma detection studies are utilized.

### (6) EXAMINATION OF IMMUNOGENICITY OF IPS CELLS

iPS cells from various sources may vary in their epigenetic alterations (Kim et al., 2010). One study suggested that iPS cells generated from fibroblasts using retroviral and episomal approaches are immunogenic in nature. (Zhao et al., 2011). However, generated iPS cells by the episomal approach were less immunogenic compared with the cells generated by the retroviral approach (Zhao et al., 2011). As reported herein, iPS cells are generated from H9c2 cells using plasmid transfection. This method generation, the number of injected cells, and the location of injection are completely different than those studies reporting immunogenicity of iPS cells (Zhao et al., 2011). Methods described herein utilize experimental groups that receive and do not receive cyclosporine.

### b. DETERMINATION OF THE EFFECTS OF INTRAMYOCARDIAL INJECTION OF DIFFERENTIATED FCC-IPS CELLS

Using an established myocardial infarction (MI) model (Singla et al., 2011), RFP-labeled differentiated FCC-iPS cells (total of 1 x 10⁵ cells) are injected in two different intramyocardial injections in the peri-infarct zone of the infarcted heart. (Singla et al., 2011). Using the same established MI model, differentiated iPS, ES cells, fibroblast reprogrammed iPS cells, H9c2 cells, FGF-8, and cell culture medium (as a control) are also injected. The effects of these injections on cardiac remodeling and regeneration are quantified.

C57BL/6 mice are treated with cyclosporine A (250-300 mg/kg) for 5 days before the cell transplantation (Menard et al., 2005; Giralt et al., 1997). The cyclosporine A dose can be altered based on the desire for an average cyclosporine serum concentration of 300-400 ng/L (Menard et al., 2005; Giralt et al., 1997). Animals are killed humanely at short-term (1, 14, and 28 days) and long-term (8, 12, and 18 weeks) time points after cell transplantation. Tissues are harvested for histological analysis and preparation of tissue homogenates.

FCC-iPS are treated as follows; FGF-8 (4 ng/mL) is added to the iPS cells in the differentiation medium (a medium which does not contain iPS cell self-renewal growth factors such as activin A, leukemia inhibitory factor and mouse embryonic fibroblast condition medium) for 48 hours. These iPS cells are considered as FGF-8 primed differentiated iPS cells. Control iPS or ES cells are cultured for 48 hours in a differentiation medium without FGF-8.

With respect to immunogenicity, any infiltration of T-cells are identified using anti-CD3 and anti-CD4 antibodies (Zhao et al., 2011). H&E stained sections are used to identify the presence of any inflammatory cells.

### c. IDENTIFICATION OF APOPTOSIS AND FIBROSIS

Heart sections are prepared (Singla et al., 2011) for immunostaining to determine apoptosis and cell proliferation. Sections are stained with a RFP or GFP antibody along with a Oct3/4 (undifferentiated cell marker on iPS or ES cells) antibody to determine total number of engrafted cells. TUNEL staining are combined with a caspase-3 antibody to establish the extent of cell death at each time point. (Singla et al., 2011; Singla et al., 2006; Singla et al., 2007). Sections are counter-stained with DAPI (Vectashield) to localize the nucleus. To determine engrafted cell apoptosis, sections are stained with RFP, Oct3/4, and TUNEL. Cell specific apoptosis within the host myocardium is determined using double-label immunostaining to detect apoptotic cells (TUNEL, Bax, and caspases) and native heart cell types. Cardiac myocytes are detected with antibodies against cardiac myosin heavy chain (MF-20 antibody, hybridoma bank), sarcomeric α-actin (Sigma), α-actinin, and cardiac troponin I (Spectral Diagnostic). (Singla et al., 2011; Singla et al., 2006). Endothelial cells are detected with factor VIII (Sigma) and Griffonia simplicifolia lectin (Sigma) antibodies. For smooth muscle (SM) cells, SM α-actin, or SM myosin heavy chain antibodies (Sigma) are used. An antibody against fibronectin (Sigma) is used to detect fibroblasts. Sections will then be stained with FITC or rhodamine conjugated secondary antibodies and counterstained with DAPI for nuclear visualization. Olympus fluorescence and confocal microscopy are used to assess apoptosis.

Heart sections are stained with Mason's trichrome and Sirius red F3BA or PSR to analyze the effect of iPS cells on gross interstitial and perivascular fibrosis. Specifically, H&E, Masson's Trichrome, and immunostained sections are used to quantify fibrosis, apoptosis, and distribution of heart cell types in the left ventricular (LV) region of the heart. (Singla et al., 2011; Singla et al., 2007). Integrated morphometry analysis (IMA) and NIH image J processing software are employed for these analyses.

### 3. DETERMINATION OF WHETHER POST-MI TRANSPLANTATION OF IPS-CM ENHANCES ATTENUATION OF ADVERSE CARDIAC REMODELING AS COMPARED TO A GROWTH FACTOR COCKTAIL

Acute MI is associated with irreversible cell loss due to apoptosis and necrosis (Anversa et al., 1997; Anversa et al., 1998; Kumar et al., 2002). Adult or embryonic stem cells have the potential to form new cardiac cells in the injured myocardium (Singla et al., 2009). Some studies suggest that an immediate release of autocrine or paracrine factors from donor stem cells decrease host myocardium remodeling (Singla et al., 2009; Gnecchi et al., 2005; Singla et al., 2010). However, there is no data available that determines the effects of secreted autocrine or paracrine factors from iPS cells in a MI model.

The data presented herein indicate that factors released from iPS cells are distinct and include anti-apoptotic and anti-fibrotic factors such as FGF-9, IL-10 and TIMP-1. Since injection of iPS-CM, which can contain numerous cytoprotective factors, does not pose a threat of teratoma formation, such an injection offers the possibility of beneficial effects on cell transplantation.

### a. EXAMINATION OF EFFECTS OF INTRAMYOCARDIAL INJECTION OF IPS-CM ON CARDIAC REMODELING POST-MI

iPS-CM from iPS-cells primed with FGF-8 and without FGF-8 is utilized (Singla et al., 2007; Singla et al., 2008). A myocardial infarction (MI) model for C57BL/6 mice having received or not having received cyclosporine A is utilized. If cyclosporine A generates no significant difference in cardiac remodeling, the use of cyclosporine A can be discontinued. Using two intramyocardial injections totaling 20 µL volume are administered post-MI for (i) FCC-iPS-CM (15x) (Singla et al., 2011; Fatma et al., 2010), (ii) a cocktail of FGF-8 (20 µL of 50 ng/mL) + FGF-9 (20 µL 50 ng/mL) + IL-10 (20 µL of 100 ng/mL (Kobbe et al., 2009)) + TIMP-1 (20 µL of 100 ng/mL (Meissbruger et al., 2011), or (iii) cell culture medium (as a control). Additional cell specific CM such ES-CM and H9c2-CM are used for comparison purposes. Subsequently, the effects on cardiac remodeling are quantified. Heart homogenates are taken and released factors are examined using Luminex technology, cytokine array, and ELISA methods (Singla et al., 2007; Singla et al., 2008; Singla et al., 2011). The levels of released factor are compared to the controls and increases are confirmed with western blotting. Heart sections are prepared for immunostaining and the degrees of apoptosis and fibrosis are evaluated. H&E, Masson's Trichrome, and Sirius red F3BA or PSR staining are used to analyze the effect of iPS cells on cell morphology and gross interstitial and perivascular fibrosis.

### b. IDENTIFICATION OF MMPS ASSOCIATED WITH FIBROSIS

SDS-PAGE and Western blot analyses are utilized to determine the protein expression of collagen types I and III (Santa Cruz Biotechnology), MMP-2 and MMP-9 (Santa Cruz Biotechnology/Cell Signaling), and TIMP-1 (Santa Cruz Biotechnology). Densitometry is used to measure band density. ELISA is performed with a kit (R & D, Minneapolis) to measure MMP-2 and MMP-9 in the LV tissue homogenates.

### c. FUNCTIONAL ASSESSMENT OF THE REGENERATED HEART

Echocardiography is an accurate noninvasive tool for the determination of quantitative characterization of post- MI heart remodeling in the mouse model. (Singla et al., 2011; Fatma et al., 2010). Echocardiography is done on all groups post-MI hearts at short-term and long-term time points. M-mode images (Singla et al., 2011; Fatma et al., 2010) in a short axis view is performed to measure LV mass, LV mass to body ratio, LV anterior and posterior wall thickness, and fractional shortening.

### 4. DETERMINATION OF WHETHER ATTENUATION OF APOPTOSIS IS MEDIATED VIA MIR-486 AND ITS PTEN AND FOXOIA TARGETS AND ASSOCIATED AKT PATHWAY.

miRNAs are small non-coding RNAs about 20-24 nucleotides in length that play a major role in the regulation of a variety of cell processes including apoptosis, hypertrophy, fibrosis, and cardiac differentiation (Care et al., 2007; Catalucci et al., 2008; Chen et al., 2006; Cimmino et al., 2005). Certain miRNAs such as miR-21, miR-133 and miR-1 are involved in various pro- and anti-apoptotic and anti-fibrotic activities in the heart (Care et al., 2007; Catalucci et al., 2008; Chen et al., 2006; Cimmino et al., 2005). In the studies described herein, miR-486 was down-regulated following MI., and that iPS cell transplantation significantly increased the levels of miR-486 following MI. While there are a few published papers describing miR-486 induction of myoblast differentiation, miR-486 involvement in cell survival in Sezary syndrome, and increased levels of miR-486 in the postnatal cardiac growth (Dey et al., 2011; Narducci et al., 2011; Small et al., 2010), there is no data regarding miR-486 levels and apoptosis following iPS cell transplantation.

### a. EXAMINATION OF MIR-486 LEVELS AND ITS TARGETS (PTEN/FOXOIA AND ASSOCIATED AKT PATHWAY)

MI hearts transplanted with primed iPS cells or CM are utilized as described above. Briefly, FCC-iPS is treated as follows; FGF-8 (4 ng/mL) is added to the iPS cells in the differentiation medium (a medium which does not contain iPS cell self-renewal growth factors such as activin A, leukemia inhibitory factor and mouse embryonic fibroblast condition medium) for 48 hours. These iPS cells are considered as FGF-8 primed differentiated iPS cells. iPS-CM from iPS-cells primed with FGF-8 and without FGF-8 is utilized.

LV are removed and miRNA is isolated using miRVANA kit (ABI).Using RT-PCR, the levels of miR-486 are ascertained. miR-486 is also confirmed with northern blotting (Dey et al., 2011; Narducci et al., 2011; Small et al., 2010). To determine the role of miR-486 on cardiac apoptosis, animals are injected with a selective inhibitor of miR-486 (locked nucleic acid, LNA-antagomir-486, 80 mg/kg) (Hatley et al., 2010; Patrick et al., 2010). To identify expression of miR-486 in the infarcted, non-infarcted, and peri-infarcted region post-MI, heart sections are stained using in situ-hybridization for miR-486. In situ-hybridization employing LNA-miR-486 specific probes (Exiqon) (Hosoda et al., 2011) is utilized. The expression of miR-486 in major heart cell types is characterized using double label immunostaining with cell specific antibodies. In brief, after in situ hybridization, sections are co-labeled with heart cell specific antibodies such as cardiac α-actin for cardiac myocytes, CD31 for endothelial cells and smooth muscle, α-actin for VSM cells as described above. Sections are counter-stained with DAPI to identify cell nuclei.

To determine whether PTEN/FOXO1a targets and associated Akt are affected at the translational level proteins are prepared from LV heart homogenates. SDS-PAGE and Western blot analyses are performed to determine the protein expression of total and phosphorylated PTEN, FOXO1a, and Akt (Cell Signaling). Densitometry is used to measure band density. Additionally, ELISA is performed with a kit (Ray Biotech Inc.) to measure total and phosphorylated PTEN, FOXO1a, and Akt.

### b. UTILIZATION OF LUCIFERASE REPORTER ASSAY FOR MIR-486 TARGETS

To confirm whether miR-486 directly binds to the 3'-UTRs of its predicted target genes, PTEN and Foxola, luciferase reporter vectors are utilized. The pCMV-Luc- 3UTR vector was constructed by sequential cloning the firefly luciferase gene (obtained from Promega) to the pcDNA 3.1 expression vector (Invitrogen) under the control of a CMV promoter. For the luciferase reporter assay, the 3'-UTR sequences for mouse PTEN (∼3.5kb in length) and Foxola (∼1 kb in length) were generated by high-fidelity PCR and then cloned downstream of the firefly luciferase stop codon. HEK 293 cells are grown in 6-well plates until they reach 80% confluency. Luciferase reporter constructs are co-transfected to the cells with a negative control vector or miR-486 expression vector in the presence or absence of a specific synthetic antagomir against miR-486. The luciferase assay is performed 48 hours after transfection using a luciferase assay kit (Promega) according to the manufacturer' instructions. Luminometer is used to quantify luciferase activity.

### D. REFERENCES

Anversa, P. et al. Apoptosis and myocardial infarction. Basic Res. Cardiol. 93 Suppl 3, 8-12 (1998).
Anversa, P. et al. Myocyte cell death and ventricular remodeling. Curr. Opin. Nephrol. Hypertens. 6, 169-176 (1997).
Balsam, L.B. et al. Haematopoietic stem cells adopt mature haematopoietic fates in ischaemic myocardium. Nature 428, 668-673 (2004).
Behfar,A. et al. Stem cell differentiation requires a paracrine pathway in the heart. FASEB J. 16, 1558-1566 (2002).
Beltrami,A.P. et al. Adult cardiac stem cells are multipotent and support myocardial regeneration. Cell 114, 763-776 (2003).
Biesecker,L.G. et al. Interleukin-6 is a component of human umbilical cord serum and stimulates hematopoiesis in embryonic stem cells in vitro. Exp. Hematol. 21, 774-778 (1993).
Care,A. et al. MicroRNA-133 controls cardiac hypertrophy. Nat. Med. 13, 613-618 (2007).
Catalucci,D. et al. MicroRNAs control gene expression: importance for cardiac development and pathophysiology 25. Ann. N. Y. Acad. Sci. 1123, 20-29 (2008).
Chang,M.Y. et al. Direct reprogramming of rat neural precursor cells and fibroblasts into pluripotent stem cells 1. PLoS. One. 5, e9838 (2010).
Chen,J.F. et al. The role of microRNA-1 and microRNA-133 in skeletal muscle proliferation and differentiation. Nat. Genet. 38, 228-233 (2006).
Cimmino,A. et al. miR-15 and miR-16 induce apoptosis by targeting BCL2 9. Proc. Natl. Acad. Sci. U. S. A 102, 13944-13949 (2005).
Damjanov,I. From stem cells to germ cell tumors and back. Verh. Dtsch. Ges. Pathol. 88, 39-44 (2004).
Dey,B.K. et al. miR-206 and -486 induce myoblast differentiation by downregulating Pax7. Mol. Cell Biol. 31, 203-214 (2011).
Dhingra,S. et al. p38 and ERK1/2 MAPKs mediate the interplay of TNF-alpha and IL-10 in regulating oxidative stress and cardiac myocyte apoptosis. Am. J. Physiol Heart Circ. Physiol 293, H3524-H3531 (2007).
Dyer,L.A. et al. The role of secondary heart field in cardiac development. Dev. Biol. 336, 137-144 (2009).
Fatma,S. et al. Factors Released from Embryonic Stem Cells Stimulate c-kit-FLK-1(+ve) Progenitor Cells and Enhance Neovascularization. Antioxid. Redox. Signal. 13, 1857-1865 (2010).
Foo, et al. Death begets failure in the heart. J Clin Invest 2005;115:565-571).
Frontini,M.J. et al. Fibroblast growth factor 9 delivery during angiogenesis produces durable, vasoresponsive microvessels wrapped by smooth muscle cells. Nat. Biotechnol. 29, 421-427 (2011).
Giralt,S. et al. Phase I trial of cyclosporine-induced autologous graft-versus-host disease in patients with multiple myeloma undergoing high-dose chemotherapy with autologous stem-cell rescue. J. Clin. Oncol. 15, 667-673 (1997).
Gnecchi,M. et al. Paracrine action accounts for marked protection of ischemic heart by Akt-modified mesenchymal stem cells. Nat. Med. 11, 367-368 (2005).
Haider & Ashraf. Bone marrow cell transplantation in clinical perspective. J Mol Cell Cardiol 2005;38:225-235.
Hatley,M.E. et al. Modulation of K-Ras-dependent lung tumorigenesis by MicroRNA-21. Cancer Cell 18, 282-293 (2010).
Hosoda,T. et al. Human cardiac stem cell differentiation is regulated by a mircrine mechanism. Circulation 123, 1287-1296 (2011).
Huang,J.Y. et al. Fibroblast growth factor 9 upregulates heme oxygenase-1 and gamma-glutamylcysteine synthetase expression to protect neurons from 1-methyl-4-phenylpyridinium toxicity. Free Radic. Biol. Med. 49, 1099-1108 (2010).
Ilagan,R. et al. FGF-8 is required for anterior heart field development 1. Development 133, 2435-2445 (2006).
Kajstura, et al. Bone marrow cells differentiate in cardiac cell lineages after infarction independently of cell fusion. Circ Res 2005;96:127-137
Kim,K. et al. Epigenetic memory in induced pluripotent stem cells. Nature 467, 285-290 (2010).
Kobbe,P. et al. IL-10 administration attenuates pulmonary neutrophil infiltration and alters pulmonary iNOS activation following hemorrhagic shock. Inflamm. Res. 58, 170-174 (2009).
Kofidis,T. et al. Stimulation of paracrine pathways with growth factors enhances embryonic stem cell engraftment and host-specific differentiation in the heart after ischemic myocardial injury. Circulation 111, 2486-2493 (2005).
Kumar,D. et al. Distinct mouse coronary anatomy and myocardial infarction consequent to ligation. Coron. Artery Dis. 16, 41-44 (2005).
Kumar,D. et al. Oxidative stress and apoptosis in heart dysfunction. Herz 27, 662-668 (2002).
Kuzmenkin,A. et al. Functional characterization of cardiomyocytes derived from murine induced pluripotent stem cells in vitro 1. FASEB J. 23, 4168-4180 (2009).
Lunde,K. et al. Intracoronary injection of mononuclear bone marrow cells in acute myocardial infarction. N. Engl. J. Med. 355, 1199-1209 (2006).
Marques,S.R. et al. Reiterative roles for FGF signaling in the establishment of size and proportion of the zebrafish heart. Dev. Biol. 321, 397-406 (2008).
Meissburger,B. et al. Tissue inhibitor of matrix metalloproteinase 1 (TIMP1) controls adipogenesis in obesity in mice and in humans. Diabetologia 54, 1468-1479 (2011).
Menard,C. et al. Transplantation of cardiac-committed mouse embryonic stem cells to infarcted sheep myocardium: a preclinical study. Lancet 366, 1005-1012 (2005).
Narducci,M.G. et al. MicroRNA profiling reveals that miR-21, miR486 and miR-214 are upregulated and involved in cell survival in Sezary syndrome. Cell Death. Dis. 2, e151 (2011).
Nelson,T.J. et al. Repair of acute myocardial infarction by human sternness factors induced pluripotent stem cells. Circulation 120, 408-416 (2009).
Nussbaum,J. et al. Transplantation of undifferentiated murine embryonic stem cells in the heart: teratoma formation and immune response. FASEB J. 21, 1345-1357 (2007).
Orlic et al., Bone marrow cells regenerate infarcted myocardium. Nature 200;410:701-705
Orlic,D. et al. Mobilized bone marrow cells repair the infarcted heart, improving function and survival. Proc. Natl. Acad. Sci. U. S. A 98, 10344-10349 (2001).
Park,I.H. et al. Disease-specific induced pluripotent stem cells 1. Cell 134, 877-886 (2008).
Park,I.H. et al. Generation of human-induced pluripotent stem cells 4. Nat. Protoc. 3, 1180-1186 (2008).
Patrick,D.M. et al. Stress-dependent cardiac remodeling occurs in the absence of microRNA-21 in mice. J. Clin. Invest 120, 3912-3916 (2010).
Reifers,F. et al. Induction and differentiation of the zebrafish heart requires fibroblast growth factor 8 (FGF-8/acerebellar) 1. Development 127, 225-235 (2000).
Shujia,J. et al. Stable therapeutic effects of mesenchymal stem cell-based multiple gene delivery for cardiac repair. Cardiovasc. Res. 77, 525-533 (2008).
Singla et al., Transplanted embryonic stem cells following mouse myocardial infarction inhibit apoptosis and cardiac remodeling. Am J PhysiolHeart Circ Physiol 2007;293:H1308-H1314).
Singla. Stem cells in the infarcted heart. J Cardiovasc Transl Res 2010;3:73-78)
Singla,D.K. et al. Factors released from embryonic stem cells inhibit apoptosis of H9c2 cells. Am. J. Physiol Heart Circ. Physiol 293, H1590-H1595 (2007).
Singla,D.K. et al. Enhancement by growth factors of cardiac myocyte differentiation from embryonic stem cells: A promising foundation for cardiac regeneration. Biochem. Biophys. Res. Commun. 335, 637-642 (2005).
Singla,D.K. et al. Transforming growth factor-beta2 enhances differentiation of cardiac myocytes from embryonic stem cells. Biochem. Biophys. Res. Commun. 332, 135-141 (2005).
Singla,D.K. Embryonic stem cells in cardiac repair and regeneration. Antioxid. Redox. Signal. 11, 1857-1863 (2009).
Singla,D.K. et al. Transplantation of embryonic stem cells into the infarcted mouse heart: formation of multiple cell types. J. Mol. Cell Cardiol. 40, 195-200 (2006).
Singla,D.K. Stem cells in the infarcted heart. J. Cardiovasc. Transl. Res. 3, 73-78 (2010).
Singla,D.K. et al. iPS Cells Repair and Regenerate Infarcted Myocardium. Mol. Pharm.(2011).
Singla,D.K. et al. Transplanted embryonic stem cells following mouse myocardial infarction inhibit apoptosis and cardiac remodeling. Am. J. Physiol Heart Circ. Physiol 293, H1308-H1314 (2007).
Singla,D.K. et al. Factors released from embryonic stem cells inhibit apoptosis in H9c2 cells through PI3K/Akt but not ERK pathway. Am. J. Physiol Heart Circ. Physiol 295, H907-H913 (2008).
Singla,D.K. et al. TGF-beta2 treatment enhances cytoprotective factors released from embryonic stem cells and inhibits apoptosis in infarcted myocardium. Am. J. Physiol Heart Circ. Physiol 300, H1442-H1450 (2011).
Slager,H.G. et al. Transforming growth factor-beta in the early mouse embryo: implications for the regulation of muscle formation and implantation. Dev. Genet. 14, 212-224 (1993).
Small,E.M. et al. Regulation of PI3-kinase/Akt signaling by muscle-enriched microRNA-486. Proc. Natl. Acad. Sci. U. S. A 107, 4218-4223 (2010).
Takahashi,K. et al. Induction of pluripotent stem cells from mouse embryonic and adult fibroblast cultures by defined factors 3. Cell 126, 663-676 (2006).
Tendera,M. et al. Intracoronary infusion of bone marrow-derived selected CD34+CXCR4+ cells and non-selected mononuclear cells in patients with acute STEMI and reduced left ventricular ejection fraction: results of randomized, multicentre Myocardial Regeneration by Intracoronary Infusion of Selected Population of Stem Cells in Acute Myocardial Infarction (REGENT) Trial 1. Eur. Heart J. 30, 1313-1321 (2009).
Thomson,J.A. et al. Embryonic stem cell lines derived from human blastocysts. Science 282, 1145-1147 (1998).
Wiles,M.V. et al. Multiple hematopoietic lineages develop from embryonic stem (ES) cells in culture. Development 111, 259-267 (1991).
Yun,Y.R. et al. Fibroblast growth factors: biology, function, and application for tissue regeneration. J. Tissue Eng 2010, 218142 (2010).
Zhang,J. et al. Functional cardiomyocytes derived from human induced pluripotent stem cells 2. Circ. Res. 104, e30-e41 (2009).
Zhao,T. et al. Immunogenicity of induced pluripotent stem cells. Nature(2011).

## Claims

1. A composition comprising fibroblast growth factor-8 (FGF-8) and/or fibroblast growth factor-8 and fibroblast growth factor-9 (FGF-9) for use in a method of regenerative medicine for enhancing cardiac tissue/cell regeneration or attenuating adverse cardiac tissue or cell remodeling in a subject.

2. The composition for use of claim 1, wherein the composition further comprises between 5,000 and 500,000 FGF-8 primed induced pluripotent stem cells.

3. The composition for use of claim 2, wherein the composition comprises 100,000 FGF-8 primed induced pluripotent stem cells, or less than 100,000 FGF-8 primed induced pluripotent stem cells, or more than 100,000 FGF-8 primed induced pluripotent stem cells.

4. The composition for use of any of the preceding claims, wherein the use comprises inducing or enhancing differentiation of induced pluripotent stem cells into cardiac myocytes.

5. The composition for use of any of the preceding claims, further comprising one or more immunosuppressive drugs, wherein the one or more immunosuppressive drugs comprise corticosteroids, calcineurin inhibitors, anti-proliferatives, and mTOR inhibitors.

6. The composition for use of any of the preceding claims, wherein the induced pluripotent stem cells are from an autologous, allogeneic, or syngeneic source.

7. The composition for use of any of claims 1 to 4, wherein the induced pluripotent stem cells are from fibroblast cells or from H9c2 cells.

8. The composition for use of claim 7, wherein the cells are transfected with at least one sternness factor.

9. The composition for use of claim 8, wherein the at least one sternness factor is c-myc, oct 3/4, Klf4, nanog, or Sox2, or a combination thereof.

10. The composition for use of any of the preceding claims, wherein the use comprises enhancing cardiac tissue and/or cell regeneration following myocardial infarction, or attenuating adverse cardiac tissue and/or cell remodeling dysfunction following myocardial infarction.

11. The composition for use of claim 10, wherein enhancing cardiac tissue and/or cell regeneration comprises enhancing cell engraftment, cell proliferation, or cell differentiation, or improving left ventricular function, improving fractional shortening, improving ejection fraction, reducing end-diastolic volume, decreasing left ventricular mass, normalizing of heart geometry, or a combination thereof, or
wherein attenuating adverse cardiac tissue and/or cell remodeling comprises inhibiting fibrosis and/or fibrosis-related mechanisms, inhibiting or decreasing necrosis, inhibiting or decreasing apoptosis and/or apoptosis-related mechanisms, or a combination thereof.

12. The composition for use of any of the preceding claims, wherein the use comprises repeated administration of the composition.

13. The composition for use of any of the preceding claims, wherein the composition is to be administered to the subject prior to, during, and/or following myocardial infarction.

14. The composition for use of claim 13, wherein the composition is to be administered to the subject within 10, 15, 20, 25, 30, or more minutes following myocardial infarction, or within 1, 2, 6, 12, 18, 24, or more hours following cardiac dysfunction.

## Patentansprüche

1. Zusammensetzung, aufweisend Fibroblasten-Wachstumsfaktor-8 (FGF-8) und/oder Fibroblasten-Wachstumsfaktor-8 und Fibroblasten-Wachstumsfaktor-9 (FGF-9) zur Anwendung in einem Verfahren der regenerativen Medizin zur Verbesserung der Regeneration von kardialem(n) Gewebe/Zellen oder zum Abschwächen von nachteiligem(n) Herzgewebe oder Zellveränderungen in einem Subjekt.

2. Zusammensetzung zur Anwendung nach Anspruch 1, wobei die Zusammensetzung weiterhin zwischen 5.000 und 500.000 mit FGF-8 vorbereitete induzierte pluripotente Stammzellen aufweist.

3. Zusammensetzung zur Anwendung nach Anspruch 2, wobei die Zusammensetzung 100.000 mit FGF-8 vorbereitete induzierte pluripotente Stammzellen oder weniger als 100.000 mit FGF-8 vorbereitete induzierte pluripotente Stammzellen oder mehr als 100.000 mit FGF-8 vorbereitete induzierte pluripotente Stammzellen aufweist.

4. Zusammensetzung zur Anwendung nach einem der vorangehenden Ansprüche, wobei die Anwendung aufweist, die Differenzierung von induzierten pluripotenten Stammzellen in kardiale Myozyten zu induzieren oder zu verstärken.

5. Zusammensetzung zur Anwendung nach einem der vorangehenden Ansprüche, weiterhin ein oder mehrere immunsuppressive Medikamente aufweisend, wobei das eine oder die mehreren immunsuppressiven Medikamente Corticosteroide, Calcineurininhibitoren, Antiproliferativmittel und mTOR-Inhibitoren aufweisen.

6. Zusammensetzung zur Anwendung nach einem der vorangehenden Ansprüche, wobei die induzierten pluripotenten Stammzellen aus einer autologen, allogenen oder syngenen Quelle stammen.

7. Zusammensetzung zur Anwendung nach einem der Ansprüche 1 bis 4, wobei die induzierten pluripotenten Stammzellen aus Fibroblastenzellen oder aus H9c2-Zellen stammen.

8. Zusammensetzung zur Anwendung nach Anspruch 7, wobei die Zellen mit wenigstens einem Stemnessfaktor transfiziert werden.

9. Zusammensetzung zur Anwendung nach Anspruch 8, wobei der wenigstens eine Stemnessfaktor c-myc, oct 3/4, Klf4, nanog oder Sox2 oder eine Kombination davon ist.

10. Zusammensetzung zur Anwendung nach einem der vorangehenden Ansprüche, wobei die Anwendung die Verbesserung der Regeneration von kardialem Gewebe und/oder kardialen Zellen oder das Abschwächen von nachteiligem Herzgewebe und/oder Zellveränderungs-Dysfunktion nach einem Myokardinfarkt aufweist.

11. Zusammensetzung zur Anwendung nach Anspruch 10, wobei die Verbesserung der Regeneration von kardialem Gewebe und/oder kardialen Zellen die Verbesserung der Zelltransplantation, Zellproliferation oder Zelldifferenzierung oder die Verbesserung der linksventrikulären Funktion, die Verbesserung des Fractional Shortening (Verkürzung des Herzens während des Pumpvorganges), die Verbesserung der Auswurffraktion, die Verringerung des enddiastolischen Volumens, die Verringerung der linksventrikulären Masse, die Normalisierung der Herzgeometrie oder eine Kombination davon aufweist, oder
wobei das Abschwächen von nachteiligem Herzgewebe und/oder Zellveränderungen das Hemmen von Fibrose und/oder Fibrose-bezogenen Mechanismen, das Hemmen oder Verringern von Nekrose, das Hemmen oder Verringern von Apoptose und/oder Apoptose-bezogenen Mechanismen oder einer Kombination davon aufweist.

12. Zusammensetzung zur Anwendung nach einem der vorangehenden Ansprüche, wobei die Anwendung die wiederholte Verabreichung der Zusammensetzung aufweist.

13. Zusammensetzung zur Anwendung nach einem der vorangehenden Ansprüche, wobei die Zusammensetzung dem Patienten vor, während und/oder nach einem Myokardinfarkt verabreicht werden soll.

14. Zusammensetzung zur Anwendung nach Anspruch 13, wobei die Zusammensetzung dem Subjekt innerhalb von 10, 15, 20, 25, 30 oder mehr Minuten nach einem Herzinfarkt oder innerhalb von 1, 2, 6, 12, 18, 24 oder mehr Stunden nach einer Herzdysfunktion zu verabreichen ist.

## Revendications

1. Composition comprenant un facteur de croissance fibroblastique 8 (FGF-8) et/ou un facteur de croissance fibroblastique 8 et un facteur de croissance fibroblastique 9 (FGF-9) à utiliser dans un procédé de médecine régénératrice pour améliorer la régénération de tissu/cellule cardiaque ou d'atténuer le remodelage nuisible de tissu ou cellule cardiaque chez un sujet.

2. Composition à utiliser selon la revendication 1, dans laquelle la composition comprend en outre entre 5 000 et 500 000 cellules-souches pluripotentes induites sensibilisées au FGF-8.

3. Composition à utiliser selon la revendication 2, dans laquelle la composition comprend 100 000 cellules-souches pluripotentes induites sensibilisées au FGF-8, ou moins de 100 000 cellules-souches pluripotentes induites sensibilisées au FGF-8, ou plus de 100 000 cellules-souches pluripotentes induites sensibilisées au FGF-8.

4. Composition à utiliser selon l'une quelconque des revendications précédentes, dans lequel l'utilisation comprend l'incitation ou l'amélioration de la différentiation de cellules-souches pluripotentes induites dans des myocytes cardiaques.

5. Composition à utiliser selon l'une quelconque des revendications précédentes, comprenant en outre un ou plusieurs médicaments immunosuppresseurs, dans laquelle le ou les plusieurs médicaments immunosuppresseurs comprennent des corticostéroïdes, des inhibiteurs de calcineurine, des anti-proliférants et des inhibiteurs de mTOR.

6. Composition à utiliser selon l'une quelconque des revendications précédentes, dans laquelle les cellules-souches pluripotentes induites proviennent d'une source autologue, allogénique, ou syngénique.

7. Composition à utiliser selon l'une quelconque des revendications 1 à 4, dans laquelle les cellules-souches pluripotentes induites proviennent de cellules fibroblastiques ou de cellules H9c2.

8. Composition à utiliser selon la revendication 7, dans laquelle les cellules sont transfectées avec au moins un facteur de caractère souche.

9. Composition à utiliser selon la revendication 8, dans laquelle l'au moins un facteur de caractère souche est c-myc, oct 3/4, Klf4, nanog, ou Sox2, ou une combinaison de ceux-ci.

10. Composition à utiliser selon l'une quelconque des revendications précédentes, dans laquelle l'utilisation comprend l'amélioration de la régénération de tissu et/ou cellule cardiaque après un infarctus myocardique, ou l'atténuation d'un dysfonctionnement de remodelage nuisible du tissu et/ou cellule cardiaque après un infarctus myocardique.

11. Composition à utiliser selon la revendication 10, dans laquelle l'amélioration de la régénération de tissu et/ou cellule cardiaque comprend l'amélioration de la prise de greffe cellulaire, de la prolifération cellulaire, ou de la différentiation cellulaire, ou l'augmentation de la fonction ventriculaire gauche, l'augmentation du raccourcissement fractionnaire, l'augmentation de la fraction d'éjection, la réduction du volume diastolique de fin, la diminution de la masse ventriculaire gauche, la normalisation de la géométrie du coeur, ou une combinaison de celles-ci, ou
dans laquelle l'atténuation d'un remodelage nuisible de tissu et/ou cellule cardiaque comprend l'inhibition de la fibrose et/ou de mécanismes concernant la fibrose, l'inhibition ou la diminution de la nécrose, l'inhibition ou la diminution de l'apoptose et/ou de mécanismes concernant l'apoptose, ou une combinaison de celles-ci.

12. Composition à utiliser selon l'une quelconque des revendications précédentes, dans laquelle l'utilisation comprend l'administration répétée de la composition.

13. Composition à utiliser selon l'une quelconque des revendications précédentes, dans laquelle la composition doit être administrée au sujet avant, pendant, et/ou après un infarctus myocardique.

14. Composition à utiliser selon la revendication 13, dans lequel la composition doit être administrée au sujet dans les 10, 15, 20, 25, 30 minutes ou plus après un infarctus myocardique, ou dans les 1, 2, 6, 12, 18, 24 heures ou plus après un dysfonctionnement cardiaque.
